(19) Europäisches Patentamt
European Patent Office
Office européen des brevets

(11) EP 3 521 445 A1

(12) **EUROPEAN PATENT APPLICATION**
published in accordance with Art. 153(4) EPC

(43) Date of publication:
**07.08.2019 Bulletin 2019/32**

(21) Application number: **17855632.0**

(22) Date of filing: **06.09.2017**

(51) Int Cl.:
***C12Q 1/68*** *(2018.01)* ***C12Q 1/02*** *(2006.01)*
***C12N 15/09*** *(2006.01)*

(86) International application number:
**PCT/JP2017/032110**

(87) International publication number:
**WO 2018/061674 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.09.2016 JP 2016193499**

(71) Applicant: **FUJIFILM Corporation**
**Tokyo 106-8620 (JP)**

(72) Inventors:
- **TSUJIMOTO Takayuki**
  **Ashigara-kami-gun**
  **Kanagawa 258-8538 (JP)**
- **ISHII Yasuyuki**
  **Ashigara-kami-gun**
  **Kanagawa 258-8538 (JP)**
- **INOUE Yuki**
  **Ashigara-kami-gun**
  **Kanagawa 258-8538 (JP)**
- **OUCHI Aya**
  **Ashigara-kami-gun**
  **Kanagawa 258-8538 (JP)**
- **NAKATANI Toshiyuki**
  **Ashigara-kami-gun**
  **Kanagawa 258-8538 (JP)**
- **ENDOH Setsu**
  **Ashigara-kami-gun**
  **Kanagawa 258-8538 (JP)**

(74) Representative: **Klunker IP**
**Patentanwälte PartG mbB**
**Destouchesstraße 68**
**80796 München (DE)**

# (54) METHOD FOR ACQUIRING BASE SEQUENCE INFORMATION ABOUT SINGLE CELLS DERIVED FROM VERTEBRATE ANIMALS

(57) Provided is a method for obtaining base sequence information of a single cell derived from a vertebrate, by which PCR amplification of an objective region can be performed uniformly and accurately.

FIG. 1

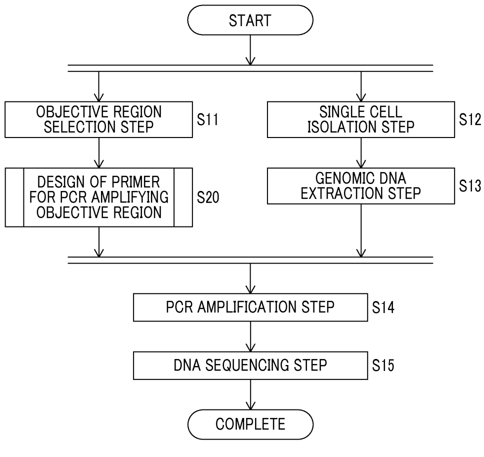


EP 3 521 445 A1

SEQUENCE LISTING

<110> Fuji Film Corp.

<120> Method for obtaining sequence information

<130> W-6025PCT

<150> JP2016-193499
<151> 2016-09-30

<160> 97

<170> PatentIn version 3.5

<210> 1
<211> 37
<212> DNA
<213> Artificial Sequence

<220>
<223> 1F

<400> 1
cgctcttccg atctctggct tggccttggg aatgtgg 37

<210> 2
<211> 37
<212> DNA
<213> Artificial Sequence

<220>
<223> 1R

<400> 2
cgctcttccg atctgacggc aatatggcca atgatgg 37

<210> 3
<211> 37
<212> DNA
<213> Artificial Sequence

<220>
<223> 2F

<400> 3
cgctcttccg atctctgctg tcagtctcag gatatgg 37

<210> 4
<211> 37
<212> DNA
<213> Artificial Sequence

<220>
<223> 2R

<400> 4
cgctcttccg atctgacgat accacagact ccgttgg 37

<210> 5
<211> 37
<212> DNA
<213> Artificial Sequence

<220>
<223> 3F

<400> 5
cgctcttccg atctctgact gctctggttg attgtgg 37

<210> 6
<211> 37
<212> DNA
<213> Artificial Sequence

<220>
<223> 3R

<400> 6
cgctcttccg atctgactgt tctactaacc ctcttgg 37

<210> 7
<211> 37
<212> DNA
<213> Artificial Sequence

<220>
<223> 4F

<400> 7
cgctcttccg atctctgttc ccggtctgcg taaatgg 37

<210> 8
<211> 37
<212> DNA
<213> Artificial Sequence

<220>
<223> 4R

<400> 8
cgctcttccg atctgacggt caaccctaag gatctgg 37

<210> 9
<211> 37
<212> DNA
<213> Artificial Sequence

<220>
<223> 5F

<400> 9
cgctcttccg atctctgtca ttctgttcat cagctgg 37

<210> 10
<211> 37
<212> DNA

<213> Artificial Sequence

<220>
<223> 5R

<400> 10
cgctcttccg atctgactaa cctgttcttc cgagtgg 37

<210> 11
<211> 37
<212> DNA
<213> Artificial Sequence

<220>
<223> 6F

<400> 11
cgctcttccg atctctgttt gcagcttgaa gggatgg 37

<210> 12
<211> 37
<212> DNA
<213> Artificial Sequence

<220>
<223> 6R

<400> 12
cgctcttccg atctgacgag catctgtttc tatgtgg 37

<210> 13
<211> 37
<212> DNA
<213> Artificial Sequence

<220>
<223> 7F

<400> 13
cgctcttccg atctctgcat cggactttgc ttgatgg 37

<210> 14
<211> 37
<212> DNA
<213> Artificial Sequence

<220>
<223> 7R

<400> 14
cgctcttccg atctgactat atgtaggccg aagttgg 37

<210> 15
<211> 37
<212> DNA
<213> Artificial Sequence

<220>

<223> 8F

<400> 15
cgctcttccg atctctggtg acgcttttta gcactgg 37

<210> 16
<211> 37
<212> DNA
<213> Artificial Sequence

<220>
<223> 8R

<400> 16
cgctcttccg atctgactct ttagagggag agattgg 37

<210> 17
<211> 37
<212> DNA
<213> Artificial Sequence

<220>
<223> 9F

<400> 17
cgctcttccg atctctgccc aacaagagaa tctatgg 37

<210> 18
<211> 37
<212> DNA
<213> Artificial Sequence

<220>
<223> 9R

<400> 18
cgctcttccg atctgactga cttcagggag cctgtgg 37

<210> 19
<211> 37
<212> DNA
<213> Artificial Sequence

<220>
<223> 10F

<400> 19
cgctcttccg atctctgtct ggggttcttc ctactgg 37

<210> 20
<211> 37
<212> DNA
<213> Artificial Sequence

<220>
<223> 10R

<400> 20

cgctcttccg atctgacctg aggaggagac tgtctgg 37

<210> 21
<211> 37
<212> DNA
<213> Artificial Sequence

<220>
<223> 11F

<400> 21
cgctcttccg atctctggcc tcgaagagag ggaatgg 37

<210> 22
<211> 37
<212> DNA
<213> Artificial Sequence

<220>
<223> 11R

<400> 22
cgctcttccg atctgacgac cacaatctct cccgtgg 37

<210> 23
<211> 37
<212> DNA
<213> Artificial Sequence

<220>
<223> 12F

<400> 23
cgctcttccg atctctgctg ggcagtgtga gaactgg 37

<210> 24
<211> 37
<212> DNA
<213> Artificial Sequence

<220>
<223> 12R

<400> 24
cgctcttccg atctgactct gaaagtgtct gttctgg 37

<210> 25
<211> 37
<212> DNA
<213> Artificial Sequence

<220>
<223> 13F

<400> 25
cgctcttccg atctctgctc atcccacaaa cagttgg 37

<210> 26
<211> 37
<212> DNA
<213> Artificial Sequence

<220>
<223> 13R

<400> 26
cgctcttccg atctgactaa tgtccccgtg tcgctgg 37

<210> 27
<211> 37
<212> DNA
<213> Artificial Sequence

<220>
<223> 14F

<400> 27
cgctcttccg atctctgaat agccagtgct gttctgg 37

<210> 28
<211> 37
<212> DNA
<213> Artificial Sequence

<220>
<223> 14R

<400> 28
cgctcttccg atctgacacc acgtagtcac tgactgg 37

<210> 29
<211> 37
<212> DNA
<213> Artificial Sequence

<220>
<223> 15F

<400> 29
cgctcttccg atctctgttc agaagctcgt caggtgg 37

<210> 30
<211> 37
<212> DNA
<213> Artificial Sequence

<220>
<223> 15R

<400> 30
cgctcttccg atctgacaag gaatgagagg cctctgg 37

<210> 31
<211> 37
<212> DNA

<213> Artificial Sequence

<220>
<223> 16F

<400> 31
cgctcttccg atctctgagg aagatgtccg ggtctgg 37

<210> 32
<211> 37
<212> DNA
<213> Artificial Sequence

<220>
<223> 16R

<400> 32
cgctcttccg atctgacatc cacctgcgga aacatgg 37

<210> 33
<211> 37
<212> DNA
<213> Artificial Sequence

<220>
<223> 17F

<400> 33
cgctcttccg atctctgccc ttaccaccat aggatgg 37

<210> 34
<211> 37
<212> DNA
<213> Artificial Sequence

<220>
<223> 17R

<400> 34
cgctcttccg atctgacttt ggttgtggtg ctgttgg 37

<210> 35
<211> 37
<212> DNA
<213> Artificial Sequence

<220>
<223> 18F

<400> 35
cgctcttccg atctctgccc gtgaagagga aatctgg 37

<210> 36
<211> 37
<212> DNA
<213> Artificial Sequence

<220>

<223> 18R

<400> 36
cgctcttccg atctgaccac aggaattgat agcgtgg 37

<210> 37
<211> 37
<212> DNA
<213> Artificial Sequence

<220>
<223> 19F

<400> 37
cgctcttccg atctctgaaa tggctgcaaa tcgatgg 37

<210> 38
<211> 37
<212> DNA
<213> Artificial Sequence

<220>
<223> 19R

<400> 38
cgctcttccg atctgacgtc ctatctcttg ctcatgg 37

<210> 39
<211> 37
<212> DNA
<213> Artificial Sequence

<220>
<223> 20F

<400> 39
cgctcttccg atctctgagc agctcaagag cgagtgg 37

<210> 40
<211> 37
<212> DNA
<213> Artificial Sequence

<220>
<223> 20R

<400> 40
cgctcttccg atctgacgtg ggtaacggca taggtgg 37

<210> 41
<211> 37
<212> DNA
<213> Artificial Sequence

<220>
<223> 21F

<400> 41

cgctcttccg atctctgata ccagttactg gcaatgg 37

<210> 42
<211> 37
<212> DNA
<213> Artificial Sequence

<220>
<223> 21R

<400> 42
cgctcttccg atctgacaca cagacagcga aagatgg 37

<210> 43
<211> 37
<212> DNA
<213> Artificial Sequence

<220>
<223> 22F

<400> 43
cgctcttccg atctctgggc aggtgtcagg cttatgg 37

<210> 44
<211> 37
<212> DNA
<213> Artificial Sequence

<220>
<223> 22R

<400> 44
cgctcttccg atctgactgg tggcctggta aaagtgg 37

<210> 45
<211> 37
<212> DNA
<213> Artificial Sequence

<220>
<223> 23F

<400> 45
cgctcttccg atctctgcag accgaaacaa gggttgg 37

<210> 46
<211> 37
<212> DNA
<213> Artificial Sequence

<220>
<223> 23R

<400> 46
cgctcttccg atctgacctt ggaaggtata gctctgg 37

<210>  47
<211>  37
<212>  DNA
<213>  Artificial Sequence

<220>
<223>  24F

<400>  47
cgctcttccg atctctgggc tgaattcttg tgactgg    37

<210>  48
<211>  37
<212>  DNA
<213>  Artificial Sequence

<220>
<223>  24R

<400>  48
cgctcttccg atctgactcc cacaacactg agcatgg    37

<210>  49
<211>  37
<212>  DNA
<213>  Artificial Sequence

<220>
<223>  25F

<400>  49
cgctcttccg atctctgtgt tatgcttggg tgaatgg    37

<210>  50
<211>  37
<212>  DNA
<213>  Artificial Sequence

<220>
<223>  25R

<400>  50
cgctcttccg atctgactac agtgagagag agcttgg    37

<210>  51
<211>  69
<212>  DNA
<213>  Artificial Sequence

<220>
<223>  D501-F

<400>  51
aatgatacgg cgaccaccga gatctacact atagccttct ttccctacac gacgctcttc    60

cgatctctg    69

<210>  52

<211> 69
<212> DNA
<213> Artificial Sequence

<220>
<223> D701-R

<400> 52
caagcagaag acggcatacg agatcgagta atgtgactgg agttcagacg tgtgctcttc 60

cgatctgac 69

<210> 53
<211> 69
<212> DNA
<213> Artificial Sequence

<220>
<223> D702-R

<400> 53
caagcagaag acggcatacg agattctccg gagtgactgg agttcagacg tgtgctcttc 60

cgatctgac 69

<210> 54
<211> 69
<212> DNA
<213> Artificial Sequence

<220>
<223> D703-R

<400> 54
caagcagaag acggcatacg agataatgag cggtgactgg agttcagacg tgtgctcttc 60

cgatctgac 69

<210> 55
<211> 69
<212> DNA
<213> Artificial Sequence

<220>
<223> D704-R

<400> 55
caagcagaag acggcatacg agatggaatc tcgtgactgg agttcagacg tgtgctcttc 60

cgatctgac 69

<210> 56
<211> 69
<212> DNA
<213> Artificial Sequence

<220>
<223> D705-R

<400> 56
caagcagaag acggcatacg agatttctga atgtgactgg agttcagacg tgtgctcttc 60

cgatctgac 69

<210> 57
<211> 69
<212> DNA
<213> Artificial Sequence

<220>
<223> D706-R

<400> 57
caagcagaag acggcatacg agatacgaat tcgtgactgg agttcagacg tgtgctcttc 60

cgatctgac 69

<210> 58
<211> 42
<212> DNA
<213> Artificial Sequence

<220>
<223> ALK_1F

<400> 58
cgctcttccg atctctgggt gtcaattact gcaggaagtg tg 42

<210> 59
<211> 38
<212> DNA
<213> Artificial Sequence

<220>
<223> ALK_1R

<400> 59
cgctcttccg atctgacgct gccagaaact gcctcttg 38

<210> 60
<211> 41
<212> DNA
<213> Artificial Sequence

<220>
<223> ALK_2F

<400> 60
cgctcttccg atctctgcac aacaactgca gcaaagactg g 41

<210> 61
<211> 42
<212> DNA
<213> Artificial Sequence

<220>
<223> ALK_2R

<400> 61
cgctcttccg atctgactgt agctgctgaa aatgtaactt tg 42

<210> 62
<211> 42
<212> DNA
<213> Artificial Sequence

<220>
<223> ALK_3F

<400> 62
cgctcttccg atctctgggt tgttccattc tggtaagaag tg 42

<210> 63
<211> 39
<212> DNA
<213> Artificial Sequence

<220>
<223> ALK_3R

<400> 63
cgctcttccg atctgacggt aagaagtggc tcactcttg 39

<210> 64
<211> 41
<212> DNA
<213> Artificial Sequence

<220>
<223> FGFR3_1F

<400> 64
cgctcttccg atctctgaca aaaacatcat caacctgctg g 41

<210> 65
<211> 40
<212> DNA
<213> Artificial Sequence

<220>
<223> FGFR3_1R

<400> 65
cgctcttccg atctgacagg acaccaggtc cttgaaggtg 40

<210> 66
<211> 41
<212> DNA
<213> Artificial Sequence

<220>
<223> FGFR3_2F

<400> 66
cgctcttccg atctctggag tacttggcct cccagaaggt g 41

<210> 67
<211> 36
<212> DNA
<213> Artificial Sequence

<220>
<223> FGFR3_2R

<400> 67
cgctcttccg atctgacgtg tgggaaggcg gtgttg 36

<210> 68
<211> 38
<212> DNA
<213> Artificial Sequence

<220>
<223> FGFR3_3F

<400> 68
cgctcttccg atctctgaga gctcaggctt caggggtg 38

<210> 69
<211> 39
<212> DNA
<213> Artificial Sequence

<220>
<223> FGFR3_3R

<400> 69
cgctcttccg atctgactca caggtcgtgt gtgcagttg 39

<210> 70
<211> 42
<212> DNA
<213> Artificial Sequence

<220>
<223> PDGFRA_1F

<400> 70
cgctcttccg atctctggca tagcaaccta gttcagtgct tg 42

<210> 71
<211> 42
<212> DNA
<213> Artificial Sequence

<220>
<223> PDGFRA_1R

<400> 71
cgctcttccg atctgactta ccaagcacta gtccatctct tg 42

<210> 72

<211> 42
<212> DNA
<213> Artificial Sequence

<220>
<223> PDGFRA_2F

<400> 72
cgctcttccg atctctgcct tgaattgatg gaagctcatt gg 42

<210> 73
<211> 42
<212> DNA
<213> Artificial Sequence

<220>
<223> PDGFRA_2R

<400> 73
cgctcttccg atctgaccca aagatatcca gctctttctt tg 42

<210> 74
<211> 42
<212> DNA
<213> Artificial Sequence

<220>
<223> PDGFRA_3F

<400> 74
cgctcttccg atctctgctt ctctagagct ttctctctgt tg 42

<210> 75
<211> 39
<212> DNA
<213> Artificial Sequence

<220>
<223> PDGFRA_3R

<400> 75
cgctcttccg atctgacagc ctgaccagtg agggaagtg 39

<210> 76
<211> 42
<212> DNA
<213> Artificial Sequence

<220>
<223> KIT_1F

<400> 76
cgctcttccg atctctgacc ctgttcactc ctttgctgat tg 42

<210> 77
<211> 42
<212> DNA
<213> Artificial Sequence

<220>
<223> cgctcttccgatctgacCCCATTTGTGATCATAAGGAAGTTG

<400> 77
cgctcttccg atctgacccc atttgtgatc ataaggaagt tg 42

<210> 78
<211> 42
<212> DNA
<213> Artificial Sequence

<220>
<223> KIT_2F

<400> 78
cgctcttccg atctctgaga aattcaggtt aaaagaggct tg 42

<210> 79
<211> 41
<212> DNA
<213> Artificial Sequence

<220>
<223> KIT_2R

<400> 79
cgctcttccg atctgacctt ctgcatgatc ttcctgcttt g 41

<210> 80
<211> 42
<212> DNA
<213> Artificial Sequence

<220>
<223> KIT_3F

<400> 80
cgctcttccg atctctggaa catcattcaa ggcgtacttt tg 42

<210> 81
<211> 40
<212> DNA
<213> Artificial Sequence

<220>
<223> KIT_3R

<400> 81
cgctcttccg atctgaccag gactgtcaag cagagaatgg 40

<210> 82
<211> 37
<212> DNA
<213> Artificial Sequence

<220>
<223> FGFR4_1F

<400> 82
cgctcttccg atctctgcat gctccctcgt gcagttg 37

<210> 83
<211> 37
<212> DNA
<213> Artificial Sequence

<220>
<223> FGFR4_1R

<400> 83
cgctcttccg atctgacact cccgcaggtt tcccttg 37

<210> 84
<211> 34
<212> DNA
<213> Artificial Sequence

<220>
<223> FGFR4_2F

<400> 84
cgctcttccg atctctgcac gggccgttag ggtg 34

<210> 85
<211> 38
<212> DNA
<213> Artificial Sequence

<220>
<223> FGFR4_2R

<400> 85
cgctcttccg atctgacaat gccacaggcc tgagagtg 38

<210> 86
<211> 42
<212> DNA
<213> Artificial Sequence

<220>
<223> EGFR_1F

<400> 86
cgctcttccg atctctgaaa cgtccctgtg ctaggtcttt tg 42

<210> 87
<211> 42
<212> DNA
<213> Artificial Sequence

<220>
<223> EGFR_1R

<400> 87
cgctcttccg atctgacgta ctgggagcca atattgtctt tg 42

<210> 88
<211> 42
<212> DNA
<213> Artificial Sequence

<220>
<223> EGFR_2F

<400> 88
cgctcttccg atctctgtct gtttcagggc atgaactact tg 42

<210> 89
<211> 42
<212> DNA
<213> Artificial Sequence

<220>
<223> EGFR_2R

<400> 89
cgctcttccg atctgacgac ctaaagccac ctccttactt tg 42

<210> 90
<211> 42
<212> DNA
<213> Artificial Sequence

<220>
<223> MET_1F

<400> 90
cgctcttccg atctctggca tttttattca agaattctgt tg 42

<210> 91
<211> 37
<212> DNA
<213> Artificial Sequence

<220>
<223> MET_1R

<400> 91
cgctcttccg atctgactac cacatctgac ttggtgg 37

<210> 92
<211> 38
<212> DNA
<213> Artificial Sequence

<220>
<223> FGFR1_1F

<400> 92
cgctcttccg atctctgaga tgataagtca caggctgg 38

<210> 93

<211> 37
<212> DNA
<213> Artificial Sequence

<220>
<223> FGFR1_1R

<400> 93
cgctcttccg atctgacggt ggggtttctt tgaggtg 37

<210> 94
<211> 40
<212> DNA
<213> Artificial Sequence

<220>
<223> FGFR2_1F

<400> 94
cgctcttccg atctctgata ggagtactcc atcccgggtg 40

<210> 95
<211> 42
<212> DNA
<213> Artificial Sequence

<220>
<223> FGFR2_1R

<400> 95
cgctcttccg atctgactag agacgaggtt tcgctatgct gg 42

<210> 96
<211> 42
<212> DNA
<213> Artificial Sequence

<220>
<223> FGFR2_2F

<400> 96
cgctcttccg atctctggaa ctttaaacat gcacacaggg tg 42

<210> 97
<211> 42
<212> DNA
<213> Artificial Sequence

<220>
<223> FGFR2_2R

<400> 97
cgctcttccg atctgaccat gctgtttcaa ctaagtcttt gg 42

**Description**

**BACKGROUND OF THE INVENTION**

1. Field of the Invention

The present invention relates to a method for obtaining base sequence information of a single cell derived from a vertebrate.

2. Description of the Related Art

In human blood, cells called rare cells are present in the blood with extremely low probability, which would not exist in a case of normal individuals, but exist in pregnant women, cancer patients, and the like. As one of the rare cells, it has been known that fetal cells migrate into the mother's blood during pregnancy and circulate the maternal body with the blood. It is said that the probability of existence in the blood is that cells exist at several ratios in several mL. In a case where it is possible to reliably analyze genomic DNA in such fetal cells with good reproducibility, it is possible to realize a gene diagnosis in which there is no possibility of miscarriage and which is safe and directly analyzes fetus-derived DNA.

In addition, as another rare cell, there is a cancer cell called a circulating tumor cell (CTC). It is said that the number of existence of this cancer cell in blood is several to several tens in 10 mL of blood. In regard to CTC, it is known that an advanced cancer cell of a human individual having a tumor or cancer circulates on the flow of blood and transfers to distant organs, and it has been realized that CTC is useful as a judgment of therapeutic effects in metastatic cancer cases such as breast cancer, prostate cancer, and colon cancer, or as a prognostic predictive factor.

The clinical usefulness of CTC is that therapeutic effects of cancer chemotherapy can be evaluated much faster than diagnostic methods of the related art. In addition, it is expected that it will be possible to select the optimal treatment for each patient based on information such as biomarkers expressed in CTC, and mutations, amplification, fusion of genes, and the like.

In recent years, because it became easy to ensure the quality and quantity of base sequence data by spreading of next generation sequencing (NGS) technology, and therefore genetic analysis has become easier to carry out. Many technical difficulties of whole genome analysis are being solved by introduction of the NGS technology. However, a total base length of genomes is generally 3 billion base pairs or more in the case of the human genome, which is generally enormous, and even with NGS technology, it takes considerable cost and time to perform whole genome analysis.

On the other hand, whole genome analysis is not optimal as a means for achieving an object of detecting a genetic abnormality. This is because it is sufficient as long as only regions on genomic DNA (including not only a coding region but also a non-coding region) related to the genetic abnormality could be analyzed. Accordingly, a Polymerase Chain Reaction (PCR) technique is spreading as a technique to efficiently and precisely analyze genes by amplifying only necessary specific regions on genomic DNA and performing reading only on base sequences thereof. In particular, a method for selectively amplifying a plurality of regions by simultaneously supplying a plurality of types of primers to one PCR reaction system is called a multiplex polymerase chain reaction (PCR).

Generally, the number of regions to be simultaneously amplified by multiplex PCR cannot be set to be large. As one of the reasons thereof, a phenomenon is known in which an unnecessary amplification product called a primer-dimer is generated due to a reaction between primers, and therefore an objective region on genomic DNA cannot be efficiently amplified.

As a means for inhibiting the formation of a primer-dimer, for example, WO2004/081225A discloses a means that enables a polymerase reaction with respect to an enormous number of regions by diving a base sequence of a primer into a constant region and a variable region, disposing the same base sequence in the constant region, and limiting bases to only two types of bases which do not become complementary to each other among cytosine (C), thymidine (T), guanine (G), and adenine (A) in the variable region.

In addition, WO2008/004691A discloses that with respect to each of combinations of primers, a score indicating complementarity at 3' terminals between primers (local alignment score at the 3' terminals) is calculated, combinations of primers with low complementarity between primers are selected, and thereby reducing a possibility that primers of different targets form primer-dimers through multiplex PCR.

**SUMMARY OF THE INVENTION**

Although these improved techniques are useful techniques in a case of amplifying abundant DNA extracted from multicellular cells; however, for example, in a case where an amount of genomic deoxyribonucleic acid (DNA; ribonucleic acid) as a template of Polymerase Chain Reaction (PCR) such as single cell analysis is extremely small, it is insufficient to suppress a primer-dimer.

Accordingly, a method for obtaining base sequence information of a single cell derived from a vertebrate, which can uniformly and accurately perform PCR amplification of an objective region accurately using genomic DNA extracted from a single cell derived from the vertebrate as a template, is required.

An object of the present invention is to provide a method for obtaining base sequence information of a single cell derived from a vertebrate, by which PCR amplification of an objective region can be performed uniformly and accurately.

The inventors of the present invention conducted intensive studies in order to solve the above-mentioned problems, and as a result, have found that, in a case of PCR amplifying an objective region using a primer designed so as to reduce complementarity between primers, PCR amplification of the objective region can be carried out uniformly and accurately, and therefore have completed the present invention.

That is, the present invention provides the following [1] to [8].

[1] A method for obtaining base sequence information of a single cell derived from a vertebrate, comprising:

an objective region selection step of selecting at least one objective region for obtaining base sequence information, from regions on vertebrate genomic DNA;
a single cell isolation step of isolating a single cell from a biological sample derived from the vertebrate;
a genomic DNA extraction step of extracting genomic DNA from the single cell;
a PCR amplification step of PCR amplifying the at least one objective region by using a primer set that is designed to PCR amplify the at least one objective region and using genomic DNA extracted in the genomic DNA extraction step as a template; and
a DNA sequencing step of decoding a DNA base sequence of a PCR amplification product obtained in the PCR amplification step so as to obtain the base sequence information of the at least one objective region,
in which the objective region selection step, and steps from the single cell isolation step to the genomic DNA extraction step are performed in random order, and
in which the primer set that is designed to PCR amplify the at least one objective region is designed through a method for designing a primer set used for a polymerase chain reaction, the designing method including:

a target region selection step a) of selecting a target region from the at least one objective region;
a primer candidate base sequence generation step b) of generating at least one base sequence of a primer candidate for PCR amplifying the target region based on each base sequence in each of vicinity regions at both ends of the target region on the vertebrate genomic DNA;
a local alignment step c) of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminal of the two base sequences;
a first stage selection step d) of performing first stage selection of the base sequence of the primer candidate for PCR amplifying the target region based on the local alignment score;
a global alignment step e) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first stage selection step;
a second stage selection step f) of performing second stage selection of the base sequence of the primer candidate for PCR amplifying the target region based on the global alignment score; and
a primer employment step g) of employing the base sequence of the primer candidate which is selected in both of the first stage selection step and the second stage selection step as the base sequence of the primer for PCR amplifying the target region,
in which both steps of the local alignment step and the first stage selection step, and both steps of the global alignment step and the second stage selection step are performed in random order or at the same time.

[2] A method for obtaining base sequence information of a single cell derived from a vertebrate, comprising:

an objective region selection step of selecting at least one objective region for obtaining base sequence information, from regions on vertebrate genomic DNA;
a single cell isolation step of isolating a single cell from a biological sample derived from the vertebrate;

a genomic DNA extraction step of extracting genomic DNA from the single cell;
a PCR amplification step of PCR amplifying the at least one objective region by using a primer set that is designed to PCR amplify the at least one objective region and using genomic DNA extracted in the genomic DNA extraction step as a template; and
a DNA sequencing step of decoding a DNA base sequence of a PCR amplification product obtained in the PCR amplification step so as to obtain the base sequence information of the at least one objective region,
in which the objective region selection step, and steps from the single cell isolation step to the genomic DNA extraction step are performed in random order, and
in which the primer set that is designed to PCR amplify the at least one objective region is designed through a method for designing a primer set used for a polymerase chain reaction, the designing method including:

a first step of target region selection $a_1$) of selecting a first target region from the at least one objective region;
a first step of primer candidate base sequence generation $b_1$) of generating at least one base sequence of a primer candidate for PCR amplifying the first target region based on each base sequence in each of vicinity regions at both ends of the first target region on the vertebrate genomic DNA;
a first step of local alignment $c_1$) of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminal of the two base sequences;
a first step of first stage selection $d_1$) of performing first stage selection of the base sequence of the primer candidate for PCR amplifying the first target region based on the local alignment score;
a first step of global alignment $e_1$) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first step of first stage selection;
a first step of second stage selection $f_1$) of performing second stage selection of the base sequence of the primer candidate for PCR amplifying the first target region based on the global alignment score;
a first step of primer employment $g_1$) of employing the base sequence of the primer candidate which is selected in both of the first step of first stage selection and the first step of second stage selection as the base sequence of the primer for PCR amplifying the first target region;
a second step of target region selection $a_2$) of selecting a second target region from objective regions which have not yet been selected from the at least one objective region;
a second step of primer candidate base sequence generation $b_2$) of generating at least one base sequence of a primer candidate for PCR amplifying the second target region based on each base sequence in each of vicinity regions at both ends of the second target region on the vertebrate genomic DNA;
a second step of local alignment $c_2$) of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminal of the two base sequences;
a second step of first stage selection $d_2$) of performing first stage selection of the base sequence of the primer candidate for PCR amplifying the second target region based on the local alignment score;
a second step of global alignment $e_2$) of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed;
a second step of second stage selection $f_2$) of performing second stage selection of the base sequence of the primer candidate for PCR amplifying the second target region based on the global alignment score; and
a second step of primer employment $g_2$) of employing the base sequence of the primer candidate which is selected in both of the second step of first stage selection and the second step of second stage selection as a base sequence of a primer for PCR amplifying the second target region,

in which both steps of the first step of local alignment and the first step of first stage selection, and both steps of the first step of global alignment and the first step of second stage selection are performed in random order or at the same time,

in which both steps of the second step of local alignment and the second step of first stage selection, and both steps of the second step of global alignment and the second step of second stage selection are performed in random order or at the same time, and

in which in a case where the at least one objective region has three or more objective regions, and in case of employing a base sequence of a primer for PCR amplifying third and subsequent target regions, which have not yet been selected from the three or more objective regions, each step from the second step of target region selection to the second step of primer employment is repeated for the third and subsequent target regions.

[3] A method for obtaining base sequence information of a single cell derived from a vertebrate, comprising:

an objective region selection step of selecting at least one objective region for obtaining base sequence information, from regions on vertebrate genomic DNA;

a single cell isolation step of isolating a single cell from a biological sample derived from the vertebrate;

a genomic DNA extraction step of extracting genomic DNA from the single cell;

a PCR amplification step of PCR amplifying the at least one objective region by using a primer set that is designed to PCR amplify the at least one objective region and using genomic DNA extracted in the genomic DNA extraction step as a template; and

a DNA sequencing step of decoding a DNA base sequence of a PCR amplification product obtained in the PCR amplification step so as to obtain the base sequence information of the at least one objective region,

in which the objective region selection step, and steps from the single cell isolation step to the genomic DNA extraction step are performed in random order, and

in which the primer set that is designed to PCR amplify the at least one objective region is designed through a method for designing a primer set used for a polymerase chain reaction, the designing method including:

a target region multiple selection step a-0) of selecting a plurality of target regions from the at least one objective region;

a primer candidate base sequence multiple generation step b-0) of generating at least one base sequence of a primer candidate for PCR amplifying the plurality of target regions based on each base sequence in each of vicinity regions at both ends of the plurality of target regions on the vertebrate genomic DNA;

a first local alignment step c-1) of obtaining a local alignment score by performing pairwise local alignment on two base sequences included in each of combinations which are obtainable by selecting base sequences of two primer candidates from the base sequences of the primer candidates for PCR amplifying the first target region among the base sequences of the primer candidates generated in the primer candidate base sequence multiple generation step, under a condition that partial sequences to be compared have 3' terminal of the two base sequences;

a first first-stage selection step d-1) of performing first stage selection of the base sequence of the primer candidate for PCR amplifying the first target region based on the local alignment score;

a first global alignment step e-1) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first first-stage selection step;

a first second-stage selection step f-1) of performing second stage selection of the base sequence of the primer candidate for PCR amplifying the first target region based on the global alignment score;

a first primer employment step g-1) of employing the base sequence of the primer candidate which is selected in both of the first first-stage selection step and the first second-stage selection step as the base sequence of the primer for PCR amplifying the first target region;

a second local alignment step c-2) of obtaining a local alignment score by performing pairwise local alignment on two base sequences included in each of combinations which are combinations obtainable by selecting base sequences of two primer candidates from the base sequences of the primer candidates for PCR amplifying the second target region among the base sequences of the primer candidates generated in the primer candidate base sequence multiple generation step and base sequences of a primer already employed, and combinations obtainable by selecting a base sequence of one primer candidate and a base sequence of one primer already employed, under a condition that partial sequences to be compared have 3' terminal of the two base sequences;
a second first-stage selection step d-2) of performing first stage selection of the base sequence of the primer candidate for PCR amplifying the second target region based on the local alignment score;
a second global alignment step e-2) of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed;
a second second-stage selection step f-2) of performing second stage selection of the base sequence of the primer candidate for PCR amplifying the second target region based on the global alignment score; and
a second primer employment step g-2) of employing the base sequence of the primer candidate which is selected in both of the second first-stage selection step and the second second-stage selection step as the base sequence of the primer for PCR amplifying the second target region,
in which both steps of the first local alignment step and the first first-stage selection step, and both steps of the first global alignment step and the first second-stage selection step are performed in random order or at the same time,
in which both steps of the second local alignment step and the second first-stage selection step, and both steps of the second global alignment step and the second second-stage selection step are performed in random order or at the same time, and
in which in a case where the at least one objective region has three or more objective regions, three or more target regions are selected in the target region multiple selection step, and a base sequence of a primer candidate for PCR amplifying each of the three or more target regions is generated in the primer candidate base sequence multiple generation step, and in case of employing a base sequence of a primer for PCR amplifying third and subsequent target regions, each step from the second local alignment step to the second primer employment step is repeated for the third and subsequent target regions.

[4] The method for obtaining base sequence information of a single cell derived from a vertebrate according to any one of [1] to [3], in which the single cell is a rare cell.
[5] The method for obtaining base sequence information of a single cell derived from a vertebrate according to [4], in which the rare cell is a nucleated red blood cell derived from a fetus.
[6] The method for obtaining base sequence information of a single cell derived from a vertebrate according to [4], in which the rare cell is a cancer cell.
[7] The method for obtaining base sequence information of a single cell derived from a vertebrate according to [6], in which the cancer cell is a circulating cancer cell.
[8] The method for obtaining base sequence information of a single cell derived from a vertebrate according to any one of [1] to [3], in which the single cell is a cancer cell derived from a solid cancer of an organ.

According to the present invention, it is possible to provide a method for obtaining base sequence information of a single cell derived from a vertebrate, by which PCR amplification of an objective region can be performed uniformly and accurately.

Furthermore, according to the present invention, it is possible to obtain base sequence information from a single cell without undergoing a whole genome amplification (WGA) step.

## BRIEF DESCRIPTION OF THE DRAWINGS

Fig. 1 is a flowchart schematically showing a method for obtaining base sequence information of a single cell derived from a vertebrate of the present invention.
Fig. 2 is a flowchart illustrating a first aspect of a method for designing a primer set used for a polymerase chain reaction, which is used in the method for obtaining base sequence information of a single cell derived from a vertebrate of the present invention.
Fig. 3 is a flowchart illustrating a second aspect of the method for designing a primer set used for a polymerase chain reaction, which is used in the method for obtaining base sequence information of a single cell derived from a vertebrate of the present invention.
Fig. 4 is a flowchart illustrating a third aspect of the method for designing a primer set used for a polymerase chain reaction, which is used in the method for obtaining base sequence information of a single cell derived from a vertebrate of the present invention.

Fig. 5 is a graph showing results of Example 1. Because four cells of cell 3, cell 8, cell 18, and cell 20 show different patterns from a sample genome, and the patterns shown by these cells are similar to each other, it is considered that the cells are derived from a fetus.

Fig. 6 is a graph showing results of Example 1. All of the four cells derived from the fetus, a ratio of chromosome 18 is about 1.5 times to both chromosome 13 and chromosome 21, which indicates chromosome 18 trisomy.

## DESCRIPTION OF THE PREFERRED EMBODIMENTS

[Method for Obtaining Base Sequence Information of Single Cell Derived From Vertebrate]

A method for obtaining base sequence information of a single cell derived from a vertebrate of the embodiment of the present invention is generally a method in which genomic DNA is extracted from a single cell obtained by isolating, from the blood, nucleated red blood cells derived from a fetus present in the peripheral blood of a pregnant woman, or rare cells such as circulating cancer cells, or a single cell belonging to a cell population of a plurality of respective tumor portions of a tumor derived from a solid cancer, PCR amplification of an objective region is performed, and therefore base sequence information of the objective region is obtained.

Characteristic points of the present invention are that, in a case of amplifying an objective region on genomic DNA by a polymerase chain reaction, PCR amplification can be carried out uniformly and accurately even with a small amount of template DNA extracted from a single cell by using a primer designed to reduce complementarity between primers.

In the present invention, in a case where a numerical value range is expressed using "to," the numerical value range includes numerical values on both sides of "to." For example, "0.1 to 0.5" includes "0.1" and "0.5" within the range thereof and has the same meaning as "equal to or greater than 0.1 to equal to or smaller than 0.5." In addition, the same applies to "0.5 to 0.1." Furthermore, the same applies to those having a magnitude relation or a context.

Hereinafter, the method for obtaining base sequence information of a single cell derived from a vertebrate of the embodiment of the present invention (hereinafter referred to as "method for obtaining base sequence information of the embodiment of the present invention" in some cases) will be described in detail.

A method for obtaining base sequence information of the embodiment of the present invention includes following (1) to (5) steps, and a step of designing a primer for PCR amplifying an objective region (Fig. 1).

(1) Objective region selection step of selecting at least one objective region for obtaining base sequence information, from regions on vertebrate genomic DNA.

(2) Single cell isolation step of isolating a single cell from a biological sample derived from the vertebrate.

(3) Genomic DNA extraction step of extracting genomic DNA from the single cell.

(4) PCR amplification step of PCR amplifying the at least one objective region by using a primer set that is designed to PCR amplify the at least one objective region and using genomic DNA extracted in the genomic DNA extraction step as a template.

(5) DNA sequencing step of decoding a DNA base sequence of a PCR amplification product of the at least one objective region that is PCR amplified in the PCR amplification step so as to obtain base sequence information of the at least one objective region.

Orders of the (1) objective region selection step, and steps from the (2) single cell isolation step to the (3) genomic DNA extraction step may be changed.

In addition, in the step of designing a primer for PCR amplifying an objective region, a primer may be designed after selection of the objective region and before PCR amplification, and a primer to be used may be prepared.

The method for designing a primer set used for a polymerase chain reaction in the step of designing a primer for PCR amplifying an objective region will be separately described in detail in "Method for Designing Primer Set Used for Polymerase Chain Reaction."

<Objective Region Selection Step>

The objective region selection step is a step of selecting at least one objective region for obtaining base sequence information, from regions on vertebrate genomic DNA.

There are many regions on vertebrate genomic DNA, from which base sequence information can be obtained, but an objective region may be selected appropriately.

<<Vertebrate>>

The vertebrate is an animal with a spine, which includes mammals, birds, reptiles, amphibians, and fish. Mammals are preferable as vertebrates, and among mammals, especially human beings are preferable.

<<Regions on Genomic DNA>>

In the method for obtaining base sequence information of a single cell derived from a vertebrate of the embodiment of the present invention, "regions on genomic DNA" refers to a region on genomic DNA in which a gene region having a possibility of genetic polymorphism, a single gene disease, a multifactorial disease exists. Here, the length of a region is not particularly limited, and may be one or more bases. The regions on genomic DNA from which an objective region is selected may exist in either a gene region or a non-gene region. Here, the gene region includes: a coding region in which a gene encoding proteins, a ribosomal ribonucleic acid (RNA) gene, a transfer RNA gene, and the like exist; and a non-coding region in which an intron dividing a gene, a transcription regulatory region, a 5'-leader sequence, a 3'-trailer sequence, and the like exist. In addition, the non-gene region includes: a non-repetitive sequence such as a pseudogene, a spacer, a response element, and a replication origin; and a repetitive sequence such as a short tandem repeat and an interspersed repetitive sequence.

Examples of genetic polymorphism include single nucleotide polymorphism (SNP), single nucleotide variant (SNV), short tandem repeat polymorphism (STRP), mutation, and insertion and/or deletion (indel). The single gene disease is a disease caused by single gene abnormality. Examples of the abnormality include deletion or duplication of the gene, and/or substitution of a base in a gene, and insertion and/or deletion. A single gene that causes a single gene disease is called a "responsible gene." The multifactorial disease is a disease in which a plurality of genes are involved in the onset. In some cases, a specific combination or the like of SNP may be related thereto. These genes are called "sensitive genes" in the sense that the genes are susceptible to a disease. Cancer is a disease caused by gene mutation. Similarly to other diseases, there is hereditary (familial) cancer which is called a genetic tumor (familial tumor) or the like.

The number of regions on genomic DNA is not particularly limited. This is because regions on genomic DNA are a candidate list in a case of selecting an objective region, and it is unnecessary to perform analysis for all the regions even if a large number of regions is listed.

<<Objective Region>>

The objective region is a region selected as a target for obtaining base sequence information from the above-described regions on genomic DNA. Here, the purpose of selection is not limited to detection of genetic polymorphism, diseases, cancer, or the like related to each region, and may be detection of aneuploidy of a chromosome or the like. In addition, the number of purposes of the selection is not limited to one, and may be two or more.

The number of regions on genomic DNA to be selected as objective regions varies depending on purposes of obtaining base sequence information. The number of regions thereof is not particularly limited, but is preferably greater than or equal to 3 regions, more preferably greater than or equal to 5 regions, and even more preferably greater than or equal to 10 regions.

The purposes of obtaining base sequence information is not particularly limited. Examples of the purposes include acquisition of a fetal genetic state, examination of fetal chromosomal aneuploidy, parent-child (fathers) appraisal between fetus and father, blood relation appraisal between a fetus and a relative, or the like in a case where base sequence information is obtained from a nucleated red blood cell of a fetus; examples thereof include evaluation of a current progress status of cancer, selection of anticancer drugs, determination of effects of anticancer drugs, or the like in a case of obtaining base sequence information from circulating cancer cells; and examples thereof include detection of a genetic abnormality, selection of a treatment method, selection of anticancer drugs, evaluation of a progress state, or the like in a case of obtaining base sequence information from a single cancer cell isolated from a solid cancer.

For example, in a case of aiming to determine a gene status of a fetus, regarding a Mendelian hereditary disease in which an abnormality of a single gene becomes a cause of a disease, a causative gene to be detected is selected from an online mendelian inheritance in man (OMIM) database, and primers of several mutation sites to be examined are designed. A target gene region of genomic DNA which has been extracted from a fetal nucleated red blood cell is amplified using the designed primer set, and a base sequence of the amplification product is obtained using a sequencer. In comparison of the base sequence which has been decoded and a healthy reference genome, in a case where deletion, duplication, inversion, and/or translocation of a gene are recognized, it is expected that the fetus may have a genetic disease.

<Single Cell Isolation Step>

The single cell isolation step is a step of isolating a single cell from a biological sample derived from the vertebrate.

<<Biological Sample>>

Biological sample is not particularly limited as long it is a sample containing cells capable of extracting genomic DNA, and examples thereof include blood, solid tissue, cultured cells, and the like.

<<Single Cell>>

Examples of the single cell include rare cells whose abundance ratio to other cells present in the blood is extremely low, only one rare cell being present in a few mL of blood.
Examples of the rare cells contained in human blood include nucleated red blood cells derived from a fetus which are contained in blood collected from a pregnant mother, blood circulating tumor cells contained in blood collected from a cancer patient, or the like.

(Nucleated Red Blood Cell Derived from Fetus)

The nucleated red blood cells derived from a fetus are erythroid precursors that pass through the placenta and are present in maternal blood, and are rare cells that are present at a proportion of 1 in about $10^6$ cells in maternal blood. During maternal pregnancy, a red blood cell of a fetus may be nucleated. Because the nuclei are present in the nucleated red blood cell, fetal genomic DNA can be obtained by isolating the nucleated red blood cells derived from the fetus.
Blood collected from a pregnant mother for the purpose of isolating the nucleated red blood cells derived from a fetus may be any blood known to have the nucleated red blood cell derived from a fetus, such as maternal blood and umbilical cord blood, and from the viewpoint of minimizing the invasiveness to the pregnant mother, maternal peripheral blood is preferable.
The peripheral blood of a pregnant mother contains maternal body-derived white blood cells such as eosinophils, neutrophils, basophils, mononuclear cells, and lymphocytes; maternal body-derived mature red blood cells having no nucleus; maternal body-derived nucleated red blood cells; and blood cells such as nucleated red blood cells derived from a fetus. It has been known that fetus-derived nucleated red blood cells exist in maternal blood from about 6 weeks after pregnancy. Accordingly, in the case of isolating the nucleated red blood cells derived from a fetus, the blood to be used in the present invention is preferably peripheral blood collected from a pregnant mother after about 6 weeks after pregnancy, or a blood sample prepared from peripheral blood collected from a pregnant mother after about 6 weeks after pregnancy.

(Isolation of Nucleated Red Blood Cell Derived from Fetus)

The nucleated red blood cells derived from a fetus can be distinguished to be isolated from other cells present in the blood by analysis using optical instruments (also called "optical analysis"). The optical analysis is preferably image analysis and/or spectroscopic analysis. In order to facilitate optical analysis, it is preferable to concentrate rare cells prior to optical analysis.
For example, a method in which the nucleated red blood cells are subjected to density gradient centrifugation to be concentrate is known.
Hereinafter, the density gradient centrifugation of the nucleated red blood cells will be explained in detail.

((Concentration by Density Gradient Centrifugation of Nucleated Red Blood Cell))

The nucleated red blood cells can be separated from plasma components and other blood cells present in the blood by density gradient centrifugation. A known method may be applied to the density gradient centrifugation for separating the nucleated red blood cells. For example, the nucleated red blood cells can be fractionated and concentrated by overlaying blood diluted with a physiological salt solution on a discontinuous density gradient in which two types of media having different densities (relative densities) are layered on a centrifuge tube, and performing centrifugation.
The density of blood cells in a maternal body including fetus-derived nucleated red blood cells is disclosed in WO2012/023298A. According to this description, an assumed density of the nucleated red blood cell derived from a fetus is about 1.065 to 1.095 g/mL, the maternal blood cell density is about 1.070 to 1.120 g/mL in a case of red blood cells, about 1.090 to 1.110 g/mL in a case of eosinophils, about 1.075 to 1.100 g/mL in a case of neutrophils, about 1.070 to 1.080 g/mL in a case of basophils, about 1.060 to 1.080 g/mL in a case of lymphocytes, and about 1.060 to 1.070 g/mL in a case of mononuclear cells.

The density (relative density) of media to be stacked is set in order to separate fetus-derived nucleated red blood cells having a density of about 1.065 to 1.095 g/mL from other blood cells. The central density of fetus-derived nucleated red blood cells is about 1.080 g/mL. Therefore, in a case where two media having different densities interposing the density are made to be adjacent to and overlap each other, it is possible to collect fractions having the nucleated red blood cell derived from a fetus on an interface between the media. The density of the medium in the underlayer is 1.08 g/mL or more and is higher than the density of the medium in the upper layer, and is preferably 1.08 g/mL to 1.10 g/mL and more preferably 1.08 g/mL to 1.09 g/mL. In addition, the density of the medium in the upper layer is 1.08 g/mL or less, and is lower than the density of the medium in the underlayer, and is preferably 1.06 g/mL to 1.08 g/mL and more preferably 1.065 g/mL to 1.08 g/mL.

As an example, it is preferable to separate plasma components, eosinophils, and mononuclear cells from the desired fractions to be collected, by setting the density of the medium in the underlayer to 1.085 g/mL and the density of medium in the upper layer to 1.075 g/mL. In addition, by setting the densities of the media, it is also possible to partially separate red blood cells, neutrophils, and lymphocytes therefrom.

In the present invention, the medium of the underlayer and the medium of the upper layer may use the same type of medium or may use different types of medium, but the same types of medium are preferably used.

Examples of the media include Percoll (manufactured by GE Healthcare Bioscience) that is a silicic acid colloidal particle dispersion which is coated with polyvinylpyrrolidone and has a diameter of 15 nm to 30 nm, Ficoll-Paque (manufactured by GE Healthcare Bioscience) which is a neutral hydrophilic polymer that is rich in side chains and formed of sucrose, Histopaque (manufactured by Sigma-Aldrich Co. LLC.) containing polysucrose and sodium diatrizoate, and the like. In the present invention, it is preferable to use Percoll and/or Histopaque. A product with a density of 1.130 is commercially available as Percoll, and it is possible to prepare a density gradient by diluting with water. For histo-packing, it is possible to prepare a density gradient using a commercially available medium with a density of 1.077 and a medium with a density of 1.119 and water.

The discontinuous density gradient of the two layers is formed in, for example, a centrifuge tube as follows.

First, the medium in the underlayer in a temperature state of a freezing point or more and 14°C or lower, preferably 8°C or lower, is accommodated in the bottom portion of the centrifuge tube, or the medium in the underlayer is cooled under a temperature of 14°C or lower, preferably 8°C or lower immediately after being accommodated in the bottom portion of the centrifuge tube.

Next, the medium in the upper layer overlaps the medium in the underlayer.

((Isolation by Flow Cytometry))

In the present invention, it is preferable to concentrate the nucleated red blood cells by density gradient centrifugation and then isolate the nucleated red blood cells by flow cytometry.

Sorting by flow cytometer is performed as follows: information derived from cells of a sample liquid is obtained by a flow cytometry method, and based on the obtained information, objective cells are fractionated into containers in which wells having openings are arranged, cells fractionated into containers are captured, and based on the images, nucleated red blood cell candidate cells are determined.

Dyeing is performed before fractionating with a flow cytometer. First, a sample to be analyzed containing objective cells is prepared. The sample to be analyzed is mixed with, for example, a hemolytic agent and a fluorescently labeled antibody used for immunostaining, and incubated, and therefore cells are immunostained. A sample liquid S is prepared by immunostaining the cells. Blood cells are irradiated with laser light or the like from a light source. Fluorescent labeling by immunostaining of the blood cells is excited by irradiation with laser light, and the blood cells emit fluorescence by fluorescent labeling by immunostaining. This fluorescence intensity is detected by a detector. On the basis of detected information, cells in which ultrasound is applied to the flow cell are charged positively or negatively. In a case where cells pass through deflecting electrode plates, one cell is basically fractionated in one well of the container by attracting charged liquid droplets to one of the deflecting electrode plates.

In some cases, it is impossible to define whether the isolated nucleated red blood cell is derived from a fetus or from a maternal body (pregnant woman) depending only on isolation by flow cytometry. However, in the present invention, it is possible to discriminate the origin of the isolated nucleated red blood cell through polymorphism analysis using single nucleotide polymorphism (SNP) and/or short tandem repeat (STR) or the like, and through genotyping analysis such as checking the presence of a Y chromosome.

(Circulating Tumor Cell)

Tumor cells circulating in the blood of solid cancer patients are known and are called circulating tumor cells (CTC). CTC is a rare cell which exists at a rate of about 1 in $10^8$ to $10^9$ blood cell components.

CTC is considered to contain cells that have the ability to metastasize from the primary tumor to other sites. It is considered that, among cancer cells that invade into the blood vessels from tumor cell masses, only a few cells that have passed through the autoimmune system circulate in the blood as CTC and form metastatic lesions. It is considered that it is extremely difficult to predict the progress of this metastatic cancer, and obtaining information of CTC and obtaining accurate information on the pathological condition of cancer is advantageous for performing treatment. Although in regard to the process of the pathology of cancer, image diagnosis is performed as diagnosis by tumor marker; however, the fact is that, it is difficult to judge in timely whether a tumor activity status, that is, a dormancy status actively proliferates. Therefore, a case where pathology of cancer could be predicted by CTC examination in the peripheral blood, becomes a very effective means.

Intratumor heterogeneity is a phenomenon in which a plurality of clones with different genomes are present in the tumor, and is a cause of the resistance to treatment of cancer. Due to the intratumor heterogeneity, even in a case where treatment-sensitive clones shrink, in a case where few clones resistant to treatment remain, this clone may proliferate and recur in some cases. The intratumor heterogeneity is perceived to be caused by clonal branching in the course of cancer evolution, and it is highly likely that medicine-resistant clones are generated also in the tumor. Accordingly, in a case of treating cancer, it is very important to consider the intratumor heterogeneity and to examine the treatment method in a timely manner.

(Isolation of Circulating Tumor Cell)

As a means for obtaining circulating tumor cells (CTC), a method for separating blood cells using a filter and using of differences in size and morphology from blood cells, and concentrating the same (for example, JP2011-163830A and JP2013-042689A) is known, for example.

In addition, as a means for obtaining blood circulating tumor cells (CTC), a cell search system (manufactured by Veridex) is commercially available. The CTC examination of cases of breast cancer, prostate cancer, and colorectal cancer using this cell search system is currently approved by the US Food and Drug Administration (FDA). CTC can be obtained by using this cell search system.

As a means for obtaining CTC using a cell search system, first, by using a magnetic particle labeled with an anti-Epithelial cell adhesion molecule (EpCAM) antibody which is an epithelial cell adhesion factor, CTC cell candidates are separated from the blood and extracted. The separated cells are further reacted with a fluorescence-labeled anti-cytokeratin monoclonal antibody, and at the same time, nuclei are stained using 4',6-diamidino-2-phenylindole (DAPI) which is a DNA staining substance. In order to identify white blood cells, the separated cells are reacted with a fluorescently labeled anti-CD45 antibody. A CTC reaction liquid is transferred to a cartridge to which the magnet is fixed, and the fluorescence coloring situation of the CTC captured by the magnet is analyzed. CTC can be identified by confirming that the nucleus stained with DAPI and morphology of cells fluorescently stained with the anti-cytokeratin monoclonal antibody are not reacted to CD45 antibody.

In addition, as a means for obtaining CTC, ClearCell FX system (manufactured by Clearbridge Biomedics) is also known as a means combining a microchannel and fluid dynamics. In this device, it is possible to concentrate and recover CTC in a label free manner without using antibodies, and it is possible to recover CTC not dependent on an expression level of an EpCAM antigen.

Specifically, it is possible to recover CTC in a tube by lysing red blood cells from approximately 7 mL of blood of a patient by RBC lysis buffer (manufactured by Takara Bio Inc.), and after centrifugation, suspending a pellet in buffer, and then filling ClearCell FX chip (manufactured by Clearbridge Biomedics) therewith.

(Isolation of Cancer Cell Derived from Solid Cancer)

Cell isolation for confirmation of the intratumor heterogeneity divides a site of the primary tumor into a plurality of sites, and therefore a single cell from that site is obtained.

Examples of a method for obtaining thereof include treatment of masses of cancer cells with trypsin or trypsin-ethylenediaminetetraacetic acid (EDTA), and the like.

In addition, such as Laser Capture Microdissection (LCM) using tumor sections, examples thereof include a method for selectively collecting and recovering target cells by a laser while observing tumor sections under a microscope by using a device to which a laser irradiation device is connected to the microscope. As a tumor section to be used, it is also possible to use formalin or a paraffin block after alcohol fixation, but a tumor section derived from a frozen tumor stored in liquid nitrogen is preferable.

<Genomic DNA Extraction Step>

The genomic DNA extraction step is a step of extracting genomic DNA from the single cells.

The genomic DNA extraction from a single cell is not particularly limited, and can be performed by a known method of the related art.

For example, a commercially available genomic DNA extraction kit may be used.

In addition, it is preferable to perform proteolytic treatment at the time of the DNA extraction. Cells contain proteins and other substances in addition to nucleic acids such as DNA and RNA, and because the most basic constituent elements of chromosomes are genomic DNA and histone proteins, a success rate of the PCR amplification step can be increased by decomposition and removal of proteins.

A method for decomposing and removing proteins is not particularly limited, but it is preferable to use proteolytic enzymes such as proteinase K. Commercially available proteolytic enzyme kits and the like can also be used.

<PCR Amplification Step>

The PCR amplification step is a step of amplifying the at least one objective region through a polymerase chain reaction by using a primer set that is designed to PCR amplify the at least one objective region and using genomic DNA extracted in the genomic DNA extraction step as a template.

The primer set designed to amplify the at least one objective region is described in "Method for Designing Primer Used in Polymerase Chain Reaction" to be described later.

(Polymerase Chain Reaction)

In the polymerase chain reaction, template DNA is repeatedly replicated using DNA polymerase. The replication is started using polymerase by adding a short DNA primer hybridizing to the template DNA in a starting portion and an ending portion of a DNA base sequence to be amplified. Two chains of template double-stranded DNA are dissociated and are individually replicated every time the replication is repeated.

In multiplex PCR, it is possible to use heat-resistant DNA polymerase and a reaction buffer which are generally used in PCR. However, in some cases, each primer pair has a different temperature annealing to template DNA, and therefore, it is necessary to examine reaction conditions. For this reason, it is preferable to use heat-resistant DNA polymerase and reaction buffer which are optimized for multiplex PCR. In the present invention, it is more preferable to cause a reaction using MULTIPLEX PCR ASSAY KIT (manufactured by TAKARA BIO INC.).

The details of the method for designing a primer and a primer set will be described below. Therefore, the outline of a case of detecting aneuploidy of chromosome 13, chromosome 18, and/or chromosome 21 will be described herein as an example.

Regarding the number of objective regions, in the case of detecting aneuploidy of chromosome 13, chromosome 18, and/or chromosome 21, target regions in necessary regions in accordance with an examination are selected from DNA sequence regions specific to the above-described chromosomes, base sequences of primer candidates for amplifying each of the target regions are generated, and a primer candidate having low complementarity between primer base sequences is selected. Accordingly, the amplification properties of an objective region are significantly improved even with respect to a trace amount of genomic DNA of a single cell or the like.

Although next generation sequencer technology is rapidly evolving, a very complicated process is required for preparing a sample to be used for sequence analysis. In a case of the most widely applied genome analysis, pretreatment of a sample requires processes such as (1) DNA fragmentation, (2) DNA size selection, (3) smoothing processing of DNA terminals, (4) addition of an adaptor sequence to DNA terminals, (5) Purification of DNA, and (6) amplification of DNA, after extracting nucleic acids from the sample.

The complicated sample adjustment step requires time and labor, and it is necessary to check whether the step has been appropriately performed. In addition, bias is caused in each step. Therefore, it is necessary to reduce this bias in a case of using a sample as a diagnostic tool in a medical field in which particularly high precision and accuracy of a result is required.

In order to solve these problems, in the present invention, it is possible to perform more uniform amplification of an objective region selected for discriminating a genetic condition by performing multiplex PCR using a primer designed through a specific method to be described below. Furthermore, it is possible to more effectively collect amplification products by purifying a PCR product using magnetic beads. Contaminants such as surplus primers, deoxynucleotides (dNTPs), and enzymes remain in a PCR reaction solution. Therefore, in some cases, the remaining contaminants become an obstacle in a case of obtaining highly accurate sequence data. However, it is possible to perform sufficient purification while significantly suppressing loss of a PCR amplification product by purifying the PCR amplification product using magnetic beads. In this case, it is preferable to use a method for reliably detecting the presence or absence of a genetic abnormality of a fetus by uniformly amplifying various gene regions accurately through only a simple sample adjustment step such as (1) amplification of DNA and (2) purification of DNA even from an extremely small amount of DNA of a single cell.

Measurement of the amount of DNA amplified can be performed, for example, using NANODROP (manufactured by Thermo Fisher Scientific) which is an ultra-trace spectrophotometer for measuring the absorbance at a wavelength of 260 nm, Agilent 2100 BIOANALYZER (manufactured by Agilent Technologies) in which a laser fluorescence detection method is used, Quantus FLUOROMETER (manufactured by Promega Corporation) for quantitatively determining double-stranded DNA through a fluorescence method, or the like.
In PCR, that is, in vitro DNA synthesis, in DNA replication in cells, that is, for in vivo DNA synthesis, two or more oligonucleotides which are called primers are required for synthesizing double-stranded DNA in PCR. In some cases, a combination of primers simultaneously used in a PCR reaction system is referred to as a primer set.
PCR can be easily extended from a simple system in which a region is amplified using a pair of primers (also referred to as "primer pair") to a complex system (multiplex PCR) in which a plurality of regions are simultaneously amplified using a primer set consisting of a plurality of primer pairs.
The advantage of PCR is that it is possible to selectively amplify only a desired region from extremely long DNA molecules of human genomic DNA (3 billion base pairs). In addition, it is possible to obtain a sufficient amount of an amplification product of a desired region using an extremely trace amount of genomic DNA as a template.
In addition, another example of an advantage of PCR includes a short period of time of about 2 hours generally required for the amplification even though the period of time depends on the programs.
Still another example of the advantage of PCR is that the process is simple, and therefore, it is possible to perform the amplification using a fully automated desktop device.

(Purification of PCR Product)

Enzymes, nucleotides, salts, and other impurities coexist in a reaction liquid containing a PCR amplification product, and therefore are preferably removed. A method in which phenol, chloroform, and ethanol are used, a method for selectively adsorbing nucleic acids on a silica carrier such as a silica membrane filter in the presence of chaotropic salts, a method in which a phenomenon that nucleic acids are selectively bonded to magnetic beads modified with carboxyl groups in the presence of polyethylene glycol (PEG) is used, and the like are known as examples of the method for purifying nucleic acids.
As the preferred embodiment of the present invention, it is possible to select a method for purifying a PCR amplification product in which magnetic beads are used.
It is possible to efficiently analyze only an objective region by purifying a PCR amplification product using magnetic beads which are paramagnetic microbeads.
A method for purifying a PCR amplification product using magnetic beads will be described.
In the method for purifying the PCR amplification product using magnetic beads, it is possible to efficiently remove contaminants such as enzymes, dNTPs, PCR primers, primer-dimers, and salts by reversibly bonding a DNA fragment, which is a PCR amplification product, to the surfaces of particles of the magnetic beads, adsorbing the magnetic beads with a magnet, and separating an amplified DNA fragment and liquid from each other. The method in which magnetic beads are used has less sample loss and higher efficiency of removing contaminants compared to other purification methods. As a result, it is possible to efficiently analyze only an objective region in the DNA sequence step. The magnetic beads are commercially available and it is possible to use, for example, AMPure XP KIT (manufactured by BECKMAN COULTER), NucleoMag (manufactured by TAKARA BIO INC.), or EpiNext DNA Purification HT System (manufactured by Epigentek Group Inc.). Among them, it is preferable to perform purification using AMPure XP KIT (manufactured by BECKMAN COULTER).

<DNA Sequencing Step>

The DNA sequencing step is a step of decoding a DNA base sequence of an amplification product of the at least one objective region that is amplified in the PCR amplification step so as to obtain base sequence information of the at least one objective region.
It is desirable to use a next generation sequencer, particularly Miseq (manufactured by Illumina, Inc.) for analyzing a sequence of a PCR amplification product. In a case of sequencing a plurality of multiplex PCR products using the next generation sequencer "Miseq," it is necessary to add P5 and P7 sequences, which are used for hybridizing to a sample identification sequence (index sequence) formed of 6 to 8 bases, and an oligonucleotide sequence on the top of a Miseq flow cell, to each of the multiplex PCR products. By adding these sequences thereto, it is possible to measure up to 96 types of multiplex PCR products at a time.
It is possible to use an adapter ligation method or a PCR method as the method for adding an index sequence and P5 and P7 sequences to both terminals of the multiplex PCR products.
In addition, in a case of mixing a plurality of multiplex PCR products and measuring the plurality of multiplex PCR products using Miseq, it is desirable to quantitatively determine each PCR product accurately. It is also possible to use Agilent 2100 BIOANALYZER (manufactured by Agilent Technologies), or Quantus FLUOROMETER (manufactured by Promega KK.) as the method for quantitatively determining PCR products. However, a method for measuring the multiplex PCR products through a quantitative PCR method is more preferable. It is preferable to perform quantitative determination as the quantitative method in the present invention using KAPA Library Quantification KIT manufactured by NIPPON Genetics Co, Ltd.

As the method for analyzing sequence data obtained using Miseq, it is preferable to map the sequence data in a well-known human genome sequence using Burrows-Wheeler Aligner (BWA: Li, H., et al., "Fast and accurate short read alignment with Burrows-Wheeler transform," Bioinformatics, 2009, Vol. 25, No. 14, PP. 1754-1760; and Li, H., et al., "Fast and accurate long-read alignment with Burrows-Wheeler transform," Bioinformatics, 2010, Vol. 26, No. 5, PP. 589-595). As means for analyzing a genetic abnormality, it is preferable to analyze genetic mutation or quantitative determination of the number of chromosomes, using SAMtools (Li, Heng, et al., "The Sequence Alignment / Map format and SAMtools," Bioinformatics, 2009, Vol. 25, No. 16, PP. 2078-2079; SAM is derived from "Sequence Alignment / Map") and/or BEDTools (Quinlan, A. R., et al., "BEDTools: a flexible suite of utilities for comparing genomic features," Bioinformatics, 2010, Vol. 26, No. 6, PP. 841-842).

(Determination of Number of Times of Sequence Reading)

In the DNA sequencing step, it is desirable to measure the number of times of sequence reading.

For example, regarding DNA fragments in which fetal nucleated red blood cells are identified and which are obtained by performing PCR amplification of a target region, the amplification amount (number of times of sequence reading) of amplification product having a sequence of a region of 140 bp to 180 bp which has been previously determined can be obtained using a sequencer. Regarding a cell which has been identified as a mother-derived nucleated red blood cell, the amplification amount (number of times of sequence reading) of amplification product having a sequence of a region of 140 bp to 180 bp which has been previously determined is obtained as a standard (or a reference) using the sequencer. In a case where fetuses are in normal states, it is expected that the ratio of the amplification amount (number of times of sequence reading) of mother-derived amplification product to the amplification amount (number of times of sequence reading) of fetus-derived amplification product becomes almost 1: 1. In a case where fetuses have a disease which is trisomy derived from an amplified chromosome, it is expected that the ratio thereof becomes almost 1: 1.5 (or 2:3).

In the present invention, the proportions of the amount (number of times of sequence reading) of fetus-derived PCR amplification products to the amount (number of times of sequence reading) of mother-derived PCR amplification products which have been collected from a plurality of pregnant maternal bodies in a case where the mothers are pregnant with normal fetuses are obtained plural times, and the distribution thereof is obtained. In addition, the proportions of the amount (number of times of sequence reading) of fetus-derived amplification products to the amount (number of times of sequence reading) of mother-derived amplification products in a case where the mothers are pregnant with fetuses with trisomy are obtained, and the distribution thereof is obtained. It is also possible to set a cutoff value in a region where these two distributions do not overlap. After comparing the cutoff value which has previously been determined with a result in which the proportion of the amplification products is obtained, it is also possible to interpret inspection results that the fetuses are normal in a case where the proportion thereof is less than or equal to the cutoff value, and the fetuses have trisomy in a case where the proportion thereof is greater than or equal to the cutoff value. In a case where no results are obtained, steps may be performed again from the isolation of single cells.

In this example, whether or not isolated nucleated red blood cells are derived from a fetus may be confirmed to determine that determination of a genetic condition of a fetus-derived single cell is performed. It is known that mother-derived nucleated red blood cells and fetus-derived nucleated red blood cells coexist in nucleated red blood cells of peripheral blood collected from a pregnant maternal body. The method of the present invention can identify fetus-derived nucleated red blood cells while determining a genetic condition of a fetus. In general, a method for detecting genetic polymorphism existing in an allele is performed as a method for identifying individual differences in a gene sequence. For example, it is also possible to use STR as a type of genetic polymorphism for father-child discrimination. In addition, as a method for identifying individual differences, it is also possible to use SNPs of which a single nucleotide in a genome base sequence is mutated and which is observed at a frequency of 1% or more. In the present invention, it is also possible to identify fetal cells and maternal cells using a next generation sequencer depending on the presence or absence of cells having STR or SNPs different from each other in nucleated red blood cells. In addition, the determination on whether nucleated red blood cells are derived from a fetus separately using supplementary information for determining a genetic condition can be performed, for example, by obtaining mother-derived white blood cells and analyzing STR or SNPs in the same manner.

In addition, in a case where it is confirmed that a fetus is a male fetus through an ultrasound inspection before collecting nucleated red blood cells, it is possible to determine whether or not the nucleated red blood cells are derived from a fetus by detecting the presence or absence of the Y chromosome in the collected nucleated red blood cells. In general, a Fluorescent in situ hybridization (FISH) method using a Y chromosome-specific fluorescent probe is known as the method for detecting the presence or absence of the Y chromosome in the collected cells. For example, CEP X/Y DNA PROBE KIT (manufactured by Abbott) is used. In the present invention, it is preferable to identify that the nucleated red blood cells are derived from a male fetus by preparing a primer having a Y chromosome-specific base sequence and checking the presence or absence of amplification of the primer through PCR.

Circulating tumor cells (CTC) are perceived to contain cells having ability to transfer from a primary tumor to other sites.

In addition, it is perceived that cells that are resistant to anticancer drugs (drug-resistant ability) are also contained therein. As examples thereof, epidermal growth factor receptor (EGFR) tyrosine kinase inhibitors such as IRESSA (R) (manufactured by AstraZeneca, common name: gefitinib) and TARCEVA (R) (manufactured by Chugai Pharmaceutical Co., Ltd., common name: erlotinib hydrochloride) have been known for patients with a non-small cell lung cancer. It is known that recurrence occurs in many patients within 1 year after starting medication. It is known that the reason thereof is because a drug resistance mutation in which, in a $790^{th}$ EGFR protein, thione is replaced by methionine (T790M), occurs in a recurrent tumor. Because this mutation interferes with drug binding, it is perceived that detecting the presence or absence of a drug resistance mutation at the start of medication or after medication is important.

Along with the development of molecular targeted drugs, a well-known case is a drug resistance mutation of the EGFR gene. Similarly, by detecting a KRAS gene mutation or a BRAF gene mutation at the same time from CTC obtained from a patient, it is possible to select effective anticancer drug treatment and to avoid unnecessary treatment and diagnosis.

As a method for detecting a mutation in a gene, a method for detecting by a real time PCR has been known. As an example, therascreen EGFR mutation detection kit RGQ (manufactured by QIAGEN CORPORATION) has been used.

In addition, as a method for detecting a gene mutation at a plurality of sites using a next generation sequencer, Ion AmpliSeq Cancer Hotspot Panel v2 (manufactured by Life Technologies, Inc.) has been known but has not been used to directly detect a genetic mutation from a single cell. Furthermore, as the method for detecting a gene mutation at a plurality of sites from a solid cancer of a patient or the like using the next generation sequencer, TruSight Cancer (manufactured by Illumina Co., Ltd.) has been known but has not been used to directly detect a genetic mutation from a single cell because an amount of DNA required for using a capture method in which oligo DNA is used instead of multiplex PCR, is 50 ng.

[Method for Designing Primer Set Used for Polymerase Chain Reaction]

The method for designing a primer set used for a polymerase chain reaction, which is carried out in a method for obtaining base sequence information of a single cell derived from a vertebrate of the present invention, will be described.

<First Aspect of Method for Designing Primer Set Used for Polymerase Chain Reaction>

In the present invention, the first aspect of the method for designing a primer set used for a polymerase chain reaction includes the following steps. In the following description, Fig. 2 is appropriately referred to.

(a) Target region selection step of selecting a target region from at least one objective region (S101 in Fig. 2).
(b) Primer candidate base sequence generation step of generating at least one base sequence of a primer candidate for PCR amplifying the target region based on each base sequence in each of vicinity regions at both ends of the target region on the vertebrate genomic DNA (S102 in Fig. 2).
(c) Local alignment step of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminal of the two base sequences (S103 in Fig. 2).
(d) First stage selection step of performing first stage selection of the base sequence of the primer candidate for PCR amplifying the target region based on the local alignment score (S104 in Fig. 2).
(e) Global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first stage selection step (S105 in Fig. 2).
(f) Second stage selection step of performing second stage selection of the base sequence of the primer candidate for PCR amplifying the target region based on the global alignment score (S106 in Fig. 2).
(g) Primer employment step of employing the base sequence of the primer candidate which is selected in both of the first stage selection step and the second stage selection step as the base sequence of the primer for PCR amplifying the target region (S107 in Fig. 2).

However, among the above steps (a) to (g), both of the above steps (c) and (d) and both of the above steps (e) and (f) are performed in random order or at the same time. That is, the steps (e) and (f) may be performed after the step (c) and the step (d) are performed, or the steps (c) and (d) may be performed after the step (e) and the step (f) are performed, or the steps (c) and (d), and the steps (e) and (f) may be performed in parallel.

In a case where the steps (c) and (d) are carried out after carrying out the steps (e) and (f), the steps (e) and (c) are preferably the following steps (e') and (c'), respectively.

(e') Global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates generated in the primer candidate base sequence generation step.
(c') Local alignment step of obtaining a local alignment score by performing pairwise local alignment on two base sequences included in each of combinations which are obtainable by selecting base sequences of two primer candidates from the base sequences of the primer candidates selected in the second stage selection step, under a condition that partial sequences to be compared have 3' terminal of the two base sequences.

In addition, in a case where the step (c) and the step (d) are carried out in parallel with the step (e) and the step (f), the step (e) is preferably the following step (e').
(e') Global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates generated in the primer candidate base sequence generation step.

Furthermore, in a case of designing a primer and in a case where a base sequence of a primer candidate has been generated, a primer is designed (S108, S109, and S110 in Fig. 2) from the b) primer candidate base sequence generation step (S102 in Fig. 2) or the a) target region selection step (S101 in Fig. 2) in a case where the base sequence of the primer candidate has not been generated from the c) local alignment step (S103 in Fig. 2).

<Second Aspect of Method for Designing Primer Set Used for Polymerase Chain Reaction>

In the present invention, the second aspect, which is one of derivation forms of the method for designing a primer set used for a polymerase chain reaction in the case where at least one objective region contains two or more objective regions, includes the following steps. In the following description, Fig. 3 is appropriately referred to.

($a_1$) First step of target region selection of selecting a first target region from the at least one objective region (S201 in Fig. 3).
($b_1$) First step of primer candidate base sequence generation of generating at least one base sequence of a primer candidate for PCR amplifying the first target region based on each base sequence in each of vicinity regions at both ends of the first target region on the vertebrate genomic DNA (S202 in Fig. 2).
($c_1$) First step of local alignment of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminal of the two base sequences (S203 in Fig. 3).
($d_1$) First step of first stage selection of performing first stage selection of the base sequence of the primer candidate for PCR amplifying the first target region based on the local alignment score (S204 in Fig. 3).
($e_1$) First step of global alignment of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first step of first stage selection (S205 in Fig. 3).
($f_1$) First step of second stage selection of performing second stage selection of the base sequence of the primer candidate for PCR amplifying the first target region based on the global alignment score (S206 in Fig. 3).
($g_1$) First step of primer employment of employing the base sequence of the primer candidate which is selected in both of the first step of first stage selection and the first step of second stage selection as a base sequence of a primer for PCR amplifying the first target region (S207 in Fig. 3).

However, among the above steps ($a_1$) to ($g_1$), both of the above steps ($c_1$) and ($d_1$) and both of the above steps ($e_1$) and ($f_1$) are performed in random order or at the same time. That is, the steps ($e_1$) and ($f_1$) may be performed after the step ($c_1$) and the step ($d_1$) are performed, or the steps ($c_1$) and ($d_1$) may be performed after the step ($e_1$) and the step ($f_1$) are performed, or the steps ($c_1$) and ($d_1$), and the steps ($e_1$) and ($f_1$) may be performed in parallel.

In a case where the steps ($c_1$) and ($d_1$) are carried out after carrying out the steps ($e_1$) and ($f_1$), the steps ($e_1$) and ($c_1$) are preferably the following steps ($e_1$') and ($c_1$'), respectively.

($e_1$') First step of global alignment of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates generated in the first step of primer candidate base sequence generation.

($c_1$') First step of local alignment of obtaining a local alignment score by performing pairwise local alignment on two base sequences included in each of combinations which are obtainable by selecting base sequences of two primer candidates from the base sequences of the primer candidates selected in the first step of second stage selection, under a condition that partial sequences to be compared have 3' terminal of the two base sequences.

In addition, in a case where the step ($c_1$) and the step ($d_1$) are carried out in parallel with the step ($e_1$) and the step (f1), the step ($e_1$) is preferably the following step ($e_1$').

($e_1$') First step of global alignment of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates generated in the first step of primer candidate base sequence generation.

($a_2$) Second step of target region selection of selecting a second target region from objective regions which have not yet been selected from the at least one objective region (S211 in Fig. 3).

($b_2$) Second step of primer candidate base sequence generation of generating at least one base sequence of a primer candidate for PCR amplifying the second target region based on each base sequence in each of vicinity regions at both ends of the second target region on the vertebrate genomic DNA (S212 in Fig. 3).

($c_2$) Second step of local alignment of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminal of the two base sequences (S213 in Fig. 3).

($d_2$) Second step of first stage selection of performing first stage selection of the base sequence of the primer candidate for PCR amplifying the second target region based on the local alignment score (S214 in Fig. 3).

($e_2$) Second step of global alignment of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed (S215 in Fig. 3).

($f_2$) Second step of second stage selection of performing second stage selection of the base sequence of the primer candidate for PCR amplifying the second target region based on the global alignment score (S216 in Fig. 3).

($g_2$) Second step of primer employment of employing the base sequence of the primer candidate which is selected in both of the second step of first stage selection and the second step of second stage selection as a base sequence of a primer for PCR amplifying the second target region (S217 in Fig. 3).

However, among the above steps ($a_2$) to ($g_2$), both of the above steps ($c_2$) and (d2) and both of the above steps ($e_2$) and ($f_2$) are performed in random order or at the same time. That is, the steps ($e_2$) and ($f_2$) may be performed after the step ($c_2$) and the step ($d_2$) are performed, or the steps ($c_2$) and ($d_2$) may be performed after the step ($e_2$) and the step ($f_2$) are performed, or the steps ($c_2$) and ($d_2$), and the steps ($e_2$) and ($f_2$) may be performed in parallel.

In a case where the steps ($c_2$) and ($d_2$) are carried out after carrying out the steps ($e_2$) and ($f_2$), the steps ($e_2$) and ($c_2$) are preferably the following steps ($e_2$') and ($c_2$'), respectively.

($e_2$') Second step of global alignment of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed.

($c_2$') Second step of local alignment of obtaining a local alignment score by performing pairwise local alignment on two base sequences included in each of combinations which are combinations obtainable by selecting base sequences of two primer candidates from the base sequences of the primer candidates selected in the second step of second stage selection and base sequences of a primer already employed, and combinations obtainable by selecting a base sequence of one primer candidate and a base sequence of one primer already employed, under a condition that partial sequences to be compared have 3' terminal of the two base sequences.

In addition, in a case where the step ($c_2$) and the step ($d_2$) are carried out in parallel with the step ($e_2$) and the step ($f_2$), the step ($e_2$) is preferably the following step ($e_2$').

($e_2$') Second step of global alignment of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed.

In the case of further designing the primer, steps from the $a_2$) second step of target region selection (S211 in Fig. 3) to the $g_2$) second step of primer employment (S217 in Fig. 3) are repeated (S208 in Fig. 3). That is, in a case where the at least one objective region has three or more objective regions, and in case of employing a base sequence of a primer for PCR amplifying third and subsequent target regions, which have not yet been selected from the three or more objective regions, each step from the ($a_2$) step to the ($g_2$) step is repeated for the third and subsequent target regions.

<Third Aspect of Method for Designing Primer Set Used for Polymerase Chain Reaction>

In the present invention, the third aspect, which is one of derivation forms of the method for designing a primer set used for a polymerase chain reaction in the case where at least one objective region contains two or more objective regions, includes the following steps. In the following description, Fig. 4 is appropriately referred to.

(a-0) Target region multiple selection step of selecting a plurality of target regions from the at least one objective region (S301 in Fig. 4).

(b-0) Primer candidate base sequence multiple generation step of generating at least one base sequence of a primer candidate for PCR amplifying the plurality of target regions based on each base sequence in each of vicinity regions at both ends of the plurality of target regions on the genomic DNA of the vertebrate (S302 in Fig. 4).

(c-1) First local alignment step of obtaining a local alignment score by performing pairwise local alignment on two base sequences included in each of combinations which are obtainable by selecting base sequences of two primer candidates from the base sequences of the primer candidates for PCR amplifying the first target region among the base sequences of the primer candidates generated in the primer candidate base sequence multiple generation step, under a condition that partial sequences to be compared have 3' terminal of the two base sequences (S303 in Fig. 4).

(d-1) First first-stage selection step of performing first stage selection of the base sequence of the primer candidate for PCR amplifying the first target region based on the local alignment score (S304 in Fig. 4).

(e-1) First global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first first-stage selection step (S305 in Fig. 4).

(f-1) First second-stage selection step of performing second stage selection of the base sequence of the primer candidate for PCR amplifying the first target region based on the global alignment score (S306 in Fig. 4).

(g-1) First primer employment step of employing the base sequence of the primer candidate which is selected in both of the first first-stage selection step and the first second-stage selection step as the base sequence of the primer for PCR amplifying the first target region (S307 in Fig. 4).

However, among the above steps (c-1) to (g-1), both of the above steps (c-1) and (d-1) and both of the above steps (e-1) and (f-1) are performed in random order or at the same time. That is, the steps (e-1) and (f-1) may be performed after the step (c-1) and the step (d-1) are performed, or the steps (c-1) and (d-1) may be performed after the step (e-1) and the step (f-1) are performed, or the steps (c-1) and (d-1), and the steps (e-1) and (f-1) may be performed in parallel.
In a case where the steps (c-1) and (d-1) are carried out after carrying out the steps (e-1) and (f-1), the steps (e-1) and (c-1) are preferably the following steps (e'-1) and (c'-1), respectively.

(e'-1) First global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates for PCR amplifying the first target region among the base sequences of the primer candidates generated in the primer candidate base sequence multiple generation step.
(c'-1) First local alignment step of obtaining a local alignment score by performing pairwise local alignment on two base sequences included in each of combinations which are obtainable by selecting base sequences of two primer candidates from base sequences of primer candidates selected in the first second-stage selection step, under a condition that partial sequences to be compared have 3' terminals of the two base sequences.

In addition, in a case where the step (c-1) and the step (d-1) are carried out in parallel with the step (e-1) and the step (f-1), the step (e-1) is preferably the following step (e'-1).

(e'-1) First global alignment step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates for PCR amplifying the first target region among the base sequences of the primer candidates generated in the primer candidate base sequence multiple generation step.
(c-2) Second local alignment step of obtaining a local alignment score by performing pairwise local alignment on two base sequences included in each of combinations which are combinations obtainable by selecting base sequences of two primer candidates from base sequences of primer candidates for PCR amplifying the second target region among the base sequences of the primer candidates generated in the primer candidate base sequence multiple generation step, and base sequences of a primer already employed, and combinations obtainable by selecting a base sequence of one primer candidate and a base sequence of one primer already employed, under a condition that partial sequences to be compared have 3' terminal of the two base sequences (S313 in Fig. 4).
(d-2) Second first-stage selection step of performing first stage selection of the base sequence of the primer candidate for PCR amplifying the second target region based on the local alignment score (S314 in Fig. 4).
(e-2) Second global alignment step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed (S315 in Fig. 4).
(f-2) Second second-stage selection step of performing second stage selection of the base sequence of the primer candidate for PCR amplifying the second target region based on the global alignment score (S316 in Fig. 4).
(g-2) Second primer employment step of employing the base sequence of the primer candidate which is selected in both of the second first-stage selection step and the second second-stage selection step as the base sequence of the primer for PCR amplifying the second target region (S317 in Fig. 4).

However, among the above steps (c-2) to (g-2), both of the above steps (c-2) and (d-2) and both of the above steps (e-2) and (f-2) are performed in random order or at the same time. That is, the steps (e-2) and (f-2) may be performed after the step (c-2) and the step (d-2) are performed, or the steps (c-2) and (d-2) may be performed after the step (e-2) and the step (f-2) are performed, or the steps (c-1) and (d-1), and the steps (e-1) and (f-1) may be performed in parallel.
In a case where the steps (c-2) and (d-2) are carried out after carrying out the steps (e-2) and (f-2), the steps (e-2) and (c-2) are preferably the following steps (e'-2) and (c'-2), respectively.

(e'-2) Second global alignment step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from base sequences of primer candidates for PCR amplifying the second target region among the base sequences of the primer candidates generated in the primer candidate base sequence multiple generation step, and base sequences of a primer already employed, and combinations obtainable by selecting a base sequence of one primer candidate and a base sequence of one primer already employed.

(c'-2) Second local alignment step of obtaining a local alignment score by performing pairwise local alignment on two base sequences included in each of combinations which are combinations obtainable by selecting base sequences of two primer candidates from the base sequences of the primer candidates selected in the second second-stage selection step and base sequences of a primer already employed, and combinations obtainable by selecting a base sequence of one primer candidate and a base sequence of one primer already employed, under a condition that partial sequences to be compared have 3' terminal of the two base sequences.

In addition, in a case where the step (c-2) and the step (d-2) are carried out in parallel with the step (e-2) and the step (f-2), the step (e-2) is preferably the following step (e'-2).

(e'-2) Second global alignment step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from base sequences of primer candidates for PCR amplifying the second target region among the base sequences of the primer candidates generated in the primer candidate base sequence multiple generation step, and base sequences of a primer already employed, and combinations obtainable by selecting a base sequence of one primer candidate and a base sequence of one primer already employed.

In the case of further designing the primer, steps from the c-2) second local alignment step (S313 in Fig. 4) to the g-2) second primer employment step (S317 in Fig. 4) are repeated (S308 in Fig. 3). That is, in a case where the at least one objective region has three or more objective regions, three or more target regions are selected in the target region multiple selection step, and the base sequence of the primer candidate for PCR amplifying each of the three or more target regions is generated in the primer candidate base sequence multiple generation step, and in case of employing the base sequence of the primer for PCR amplifying third and subsequent target regions, each step from the second local alignment step to the second primer employment step is repeated for the third and subsequent target regions.

<Description of Each Step>

Hereinafter, each step included in the method for designing a primer set used for a polymerase chain reaction, which is carried out in a method for obtaining base sequence information of a single cell derived from a vertebrate of the present invention, will be described.

«Target Region Selection Step»

In the present specification, the target region selection step S101 (Fig. 2), the first step of target region selection S201 and the second step of target region selection S211 (Fig. 3), and the target region multiple selection step S301 (Fig. 4) will be collectively referred to as a "target region selection step" in some cases.

(First Aspect: Target Region Selection Step S101)

This step is shown as "target region selection" (S101) in Fig. 2.

In the first aspect, (a) target region selection step is a step of selecting a target region from an objective region.

(Second Aspect: First Step of Target Region Selection S201 and Second Step of Target Region Selection S211)

These steps are shown as "first step of target region selection" (S201) and "second step of target region selection" (S211) in Fig. 3.

In the second aspect, the ($a_1$) first step of target region selection is a step of selecting a first target region from an objective region, and the ($a_2$) second step of target region selection is a step of selecting a second target region from an objective region which has not yet been selected.

In the second aspect, objective regions are selected one by one.

(Third aspect: Target Region Multiple Selection Step S301)

This step is shown as "target region multiple selection" (S301) in Fig. 4.

In the third aspect, (a-0) target region multiple selection step is a step of selecting a plurality of target regions from an objective region.

In the third aspect, a plurality of objective regions are selected. All objective regions are preferably selected as target regions.

«Primer Candidate Base Sequence Generation Step»

In the present specification, the primer candidate base sequence generation step S102 (Fig. 2), the first step of primer candidate base sequence generation S202 and the second step of primer candidate base sequence generation S212 (Fig. 3), and the primer candidate base sequence multiple generation step S302 (Fig. 4) will be collectively referred to as a "primer candidate base sequence generation step" in some cases.

(First Aspect: Primer Candidate Base Sequence Generation Step S102)

This step is shown as "primer candidate base sequence generation" (S102) in Fig. 2.

In the first aspect, the (b) primer candidate base sequence generation step is a step of generating at least one base sequence of a primer candidate for PCR amplifying the target region based on each base sequence in each of vicinity regions at both ends of the target region on the genomic DNA.

(Second Aspect: First Step of Primer Candidate Base Sequence Generation S202 and Second Step of Primer Candidate Base Sequence Generation S212)

These steps are shown as "first step of primer candidate base sequence generation" (S202) and "second step of primer candidate base sequence generation" (S212) in Fig. 3.

In the second aspect, the ($b_1$) first step of primer candidate base sequence generation is a step of generating at least one base sequence of a primer candidate for PCR amplifying the first target region based on each base sequence in each of vicinity regions at both ends of the first target region on the genomic DNA, and the ($b_2$) second step of primer candidate base sequence generation is a step of generating at least one base sequence of a primer candidate for PCR amplifying the second target region based on each base sequence in each of vicinity regions at both ends of the second target region on the genomic DNA.

In the second aspect, with respect to one target region, generation of a base sequence of a primer candidate, selection of a primer candidate, and employment of a primer are carried out, and the same steps are repeated for the next one target region.

(Third Aspect: Primer Candidate Base Sequence Multiple Generation Step S302)

This step is shown as "primer candidate base sequence multiple generation" (S302) in Fig. 4.

In the third aspect, the (b-0) primer candidate base sequence multiple generation step is a step of generating at least one base sequence of a primer candidate for PCR amplifying the plurality of target regions based on each base sequence in each of vicinity regions at both ends of the plurality of target regions on the genomic DNA.

In the third aspect, a base sequence of a primer candidate is generated for all of the plurality of target regions, and selection and employment are repeated in subsequent steps.

(Vicinity Regions)

Each vicinity regions of the target region at both ends is collectively referred to as regions on the outside of the 5' terminal of the target region and regions on the outside of the 3' terminal of the target region. The inside of the target region is not included in the vicinity regions.

A length of the vicinity region is not particularly limited, but is preferably less than or equal to a length that can be expanded through PCR and more preferably less than or equal to the upper limit of a fragment length of DNA for which amplification is desired. A length facilitating application of concentration selection and/or sequence reading is particularly preferable. A length of the vicinity region may be appropriately changed in accordance with the type of enzyme (DNA polymerase) used for PCR. A specific length of the vicinity region is preferably about 20 to 500 bases, more preferably about 20 to 300 bases, still more preferably about 20 to 200 bases, and particularly preferably about 50 to 200 bases.

(Design Parameter of Primer)

In addition, in a case of generating a base sequence of a primer candidate, points, such as the length of a primer, the GC content (referring to a total mole percentage of guanine (G) and cytosine (C) in all nucleic acid bases), a melting temperature (which is a temperature at which 50% of double-stranded DNA is dissociated and becomes single-stranded DNA, and in which Tm is derived from a melting temperature and is referred to as "Tm value" in some cases, and the unit is "°C"), and deviation of a sequence, to be taken into consideration in a general method for designing a primer are the same.

. Length of Primer

A length of a primer (the number of nucleotides) is not particularly limited, but is preferably 15 mer to 45 mer, more preferably 20 mer to 45 mer, and still more preferably 20 mer to 30 mer. In a case where a length of a primer is within this range, it is easy to design a primer excellent in specificity and amplification efficiency. The unit "mer" is a unit in a case where a length of polynucleotide is expressed by the number of nucleotides, and 1 mer represents one nucleotide. Therefore, for example, 15 mer represents a polynucleotide consisting of 15 nucleotides.

. GC Content of Primer

A GC content of the primer is not particularly limited, but is preferably 40 mol% to 60 mol% and more preferably 45 mol% to 55 mol%. In a case where a GC content is within this range, a problem such as a decrease in the specificity and the amplification efficiency due to a high-order structure is less likely to occur.

. Tm Value of Primer

A Tm value of the primer is not particularly limited, but is preferably within a range of 50°C to 65°C and more preferably within a range of 55°C to 65°C.

A difference in the Tm value of the primer is preferably 5°C or less and more preferably 3°C or less, in a primer pair and primer set.

Tm values can be calculated using software such as OLIGO Primer Analysis Software (manufactured by Molecular Biology Insights) or Primer 3 (http://www-genome.wi.mit.edu/ftp/distribution/software/) and the like.

In addition, the Tm value can also be obtained through calculation using the following formula from the number of A's, T's, G's, and C's (which are respectively set as nA, nT, nG, and nC) in a base sequence of a primer.

$$\text{Tm value } (^\circ\text{C}) = 2(nA + nT) + 4(nC + nG)$$

The method for calculating the Tm value is not limited thereto and can be calculated through various well-known methods in the related art.

. Deviation of Base of Primer

The base sequence of a primer candidate is preferably set as a sequence in which there is no deviation of bases as a whole. For example, it is desirable to avoid a GC-rich sequence and a partial AT-rich sequence.

In addition, it is also desirable to avoid continuation of T and/or C (polypyrimidine) and continuation of A and/or G (polypurine).

. 3' Terminal of Primer

Furthermore, it is preferable that a 3' terminal base sequence avoids a GC-rich sequence or an AT-rich sequence. G or C is preferable for a 3' terminal base, but is not limited thereto.

<<Specificity-Checking Step>>

A specificity-checking step of evaluating specificity of a base sequence of a primer candidate may be performed based on sequence complementarity with respect to chromosomal DNA of a base sequence of each primer candidate which has been generated in the "primer candidate base sequence generation step."

In the specificity check, in a case where local alignment of a base sequence of chromosomal DNA and a base sequence of a primer candidate is performed and a local alignment score is less than a predetermined value, it is possible to evaluate that the complementarity of the base sequence of the primer candidate with respect to genomic DNA is low and the specificity of the base sequence of the primer candidate with respect to genomic DNA is high. Here, it is desirable to perform local alignment on also a complementary chain of chromosomal DNA. This is because chromosomal DNA is double-stranded whereas the primer is single-stranded DNA. In addition, a base sequence complementary to the base sequence of the primer candidate may be used instead of the base sequence of the primer candidate.

In addition, homology search may be performed on genomic DNA base sequence database using the base sequence of the primer candidate as a query sequence. Examples of a homology search tool include Basic Local Alignment Search Tool (BLAST) (Altschul, S. A., et al., "Basic Local Alignment Search Tool", Journal of Molecular Biology, 1990, October, Vol. 215, pp. 403-410) and FASTA (Pearson, W. R., et al., "Improved tools for biological sequence comparison", Proceedings of the National Academy of Sciences of the United States of America, National Academy of Sciences, 1988, April, Vol. 85, pp. 2444-2448). It is possible to obtain local alignment as a result of performing the homology search.

All of the scores and a threshold value of a local alignment score are not particularly limited, and can be appropriately set in accordance with the length of a base sequence of a primer candidate and/or PCR conditions, and the like. In a case of using a homology search tool, a default value of the homology search tool may be used.

For example, as the scores, it is considered that match (complementary base) = +1, mismatch (non-complementary base) = -1, and an indel (insertion and/or deletion) = -3 are employed, and the threshold value is set to be +15.

In a case where a base sequence of a primer candidate has complementarity to a base sequence at an unexpected position on chromosomal DNA but has low specificity thereto, in some cases, an artifact is amplified instead of a target region in a case where PCR is performed using a primer of the base sequence of a primer candidate. Therefore, the case where the base sequence of the primer candidate has complementarity to the base sequence at an unexpected position on genomic DNA but has low specificity thereto is excluded.

«Local Alignment Step»

In the present specification, the local alignment step S103 (Fig. 2), the first step of local alignment S203 and the second step of local alignment S213 (Fig. 3), and the first local alignment step S303 and the second local alignment step S313 (Fig. 4) will be collectively referred to as a "local alignment step" in some cases.

(First Aspect: Local Alignment Step S103)

This step is shown as "local alignment" (S103) in Fig. 2.

In the first aspect, the (c) local alignment step is a step of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminal of the two base sequences.

(Second Aspect: First Step of Local Alignment S203 and Second Step of Local Alignment S213)

These steps are shown as "first step of local alignment" (S203) and "second step of local alignment" (S213) in Fig. 3.

In the second aspect, the ($c_1$) first step of local alignment is a step of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminal of the two base sequences. The ($c_2$) second step of local alignment is a step of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminal of the two base sequences.

(Third Aspect: First Local Alignment Step S303 and Second Local Alignment Step S313)

These steps are shown as "first local alignment" (S303) and "second local alignment" (S313) in Fig. 4.

In the third aspect, the (c-1) first local alignment step is a step of obtaining a local alignment score by performing pairwise local alignment on two base sequences included in each of combinations which are obtainable by selecting base sequences of two primer candidates from the base sequences of the primer candidates for PCR amplifying the first target region which are selected from the base sequences of the primer candidates generated in the primer candidate base sequence multiple generation step, under a condition that partial sequences to be compared have 3' terminal of the two base sequences. The (c-2) second local alignment step is a step of obtaining a local alignment score by performing pairwise local alignment on two base sequences included in each of combinations which are combinations obtainable by selecting base sequences of two primer candidates from the base sequences of the primer candidates for PCR amplifying the second target region selected among the base sequences of the primer candidates generated in the primer candidate base sequence multiple generation step and base sequences of a primer already employed, and combinations obtainable by selecting a base sequence of one primer candidate and a base sequence of one primer already employed, under a condition that partial sequences to be compared have 3' terminal of the two base sequences.

(Local Alignment Method)

A combination of base sequences to be subjected to local alignment may be a combination selected while allowing overlapping, or may be a combination selected without allowing overlapping. However, in a case where formability of a primer-dimer between primers of an identical base sequence has not yet been evaluated, the combination selected while allowing overlapping is preferable.

The total number of combinations is "${}_{p}H_{2} = {}_{p+1}C_{2} = (p+1)! / 2(p-1)!$" in a case where the selection is performed while allowing overlapping, and is "${}_{p}C_{2} = p(p-1) / 2$" in a case where the selection is performed without allowing overlapping, in which the total number of base sequences for performing local alignment is set to be p.

Local alignment is alignment which is performed on a partial sequence and in which it is possible to locally check a portion with high complementarity.

However, in the present invention, the local alignment is different from local alignment usually performed on a base sequence, and is designed such that partial sequences to be subjected to comparison include the 3' terminals of both base sequences by performing local alignment under the condition that the "partial sequences to be subjected to comparison include the 3' terminals of the base sequences."

Furthermore, in the present invention, an aspect is preferable in which partial sequences to be subjected to comparison include the 3' terminals of both base sequences by performing local alignment under the condition that the "partial sequences to be subjected to comparison include the 3' terminals of the base sequences," that is, the condition that "only alignments in which a partial sequence to be subjected to comparison begins at the 3' terminal of one sequence and ends at the 3' terminal of the other sequence."

Local alignment may be performed by inserting a gap. The gap means insertion and/or deletion (indel) of a base.

In addition, in the local alignment, a case where bases are complementary to each other between base sequence pairs is regarded as a match and a case where bases are not complementary to each other therebetween is regarded as a mismatch.

Alignment is performed such that scores for each of the match, the mismatch, and the indel are given and the total score becomes a maximum. The score may be appropriately set. For example, scores may be set as in Table 1. "-" in Table 1 represents a gap (insertion and/or deletion (indel)).

[Table 1]

| | A | T | G | C | - |
|---|---|---|---|---|---|
| A | -1 | 1 | -1 | -1 | -1 |
| T | +1 | -1 | -1 | -1 | -1 |
| G | -1 | -1 | -1 | -1 | -1 |
| C | -1 | -1 | 1 | -1 | -1 |
| - | -1 | -1 | -1 | -1 | |

"-" :gap(indel)

For example, it is considered that local alignment is performed on base sequences of SEQ ID No: 1 and SEQ ID No: 2 shown in the following Table 2. Here, scores are as shown in Table 1.

[Table 2]

| | Base sequence (5'→3') |
|---|---|
| SEQ ID No: 1: | CGCTCTTCCGATCTCTGGCTTGGCCTTGGGAATGTGG |

(continued)

| | Base sequence (5'→3') |
|---|---|
| SEQ ID No: 2: | CGCTCTTCCGATCTGACGGCAATATGGCCAATGATGG |

From the base sequences of SEQ ID No: 1 and SEQ ID No: 2, a dot matrix shown in Table 3 is generated. Specifically, the base sequence of SEQ ID No: 1 is arranged from the left to the right in an orientation of 5' to 3' and the base sequence of SEQ ID No: 2 is arranged from the bottom to the top in an orientation of 5' to 3'. "·" is filled in a grid of which bases are complementary to each other, and a dot matrix shown in Table 3 is obtained.

[Table 3]

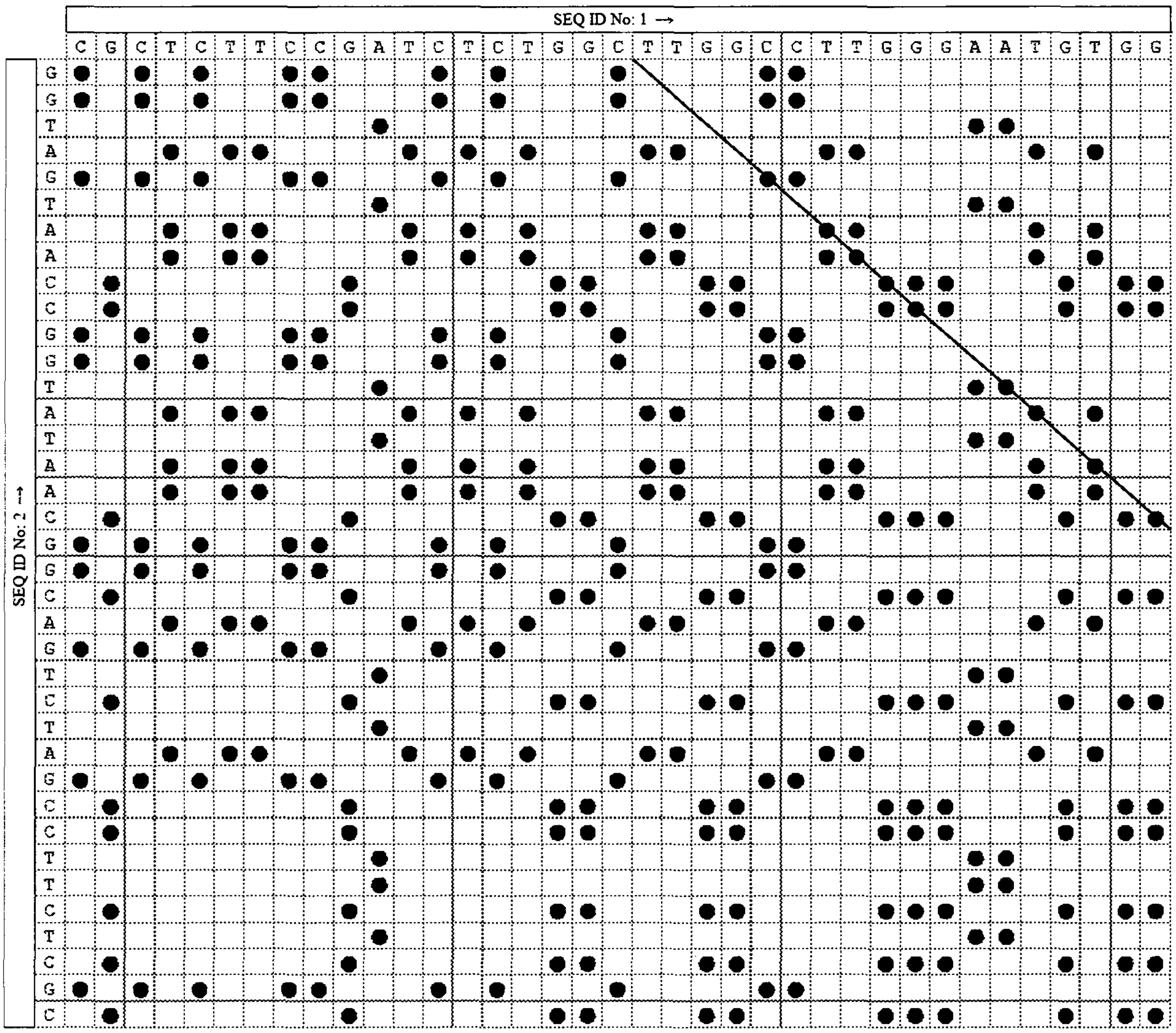


From the dot matrix shown in Table 3, Alignment (pairwise alignment) of partial sequences shown in the following Table 4 is obtained (refer to a diagonal line portion of Table 3). In Table 4, matches are represented by "1," and mismatches are represented by ":."

[Table 4]

| | | | | | | | | | | | | | | | | | | | | |
|---|---|---|---|---|---|---|---|---|---|---|---|---|---|---|---|---|---|---|---|---|
| Partial sequence from SEQ ID No: 1: | 5'- | T | T | G | G | C | C | T | T | G | G | G | A | A | T | G | T | G | G | -3' |
| | | : | : | : | : | \| | : | \| | \| | \| | \| | : | : | \| | \| | : | \| | : | \| | |
| Partial sequence from SEQ ID No: 2 | 3'- | G | G | T | A | G | T | A | A | C | C | G | G | T | A | T | A | A | C | -5' |

In this (pairwise) alignment, there are nine matches, there are eight mismatches, and no indel (gap).

Therefore, a local alignment score based on this (pairwise) alignment is (+1) × 9 + (-1) × 8 +(-1) × 0 = +1.

The alignment (pairwise alignment) can be obtained not only through the dot matrix method exemplified herein, but also through a dynamic programming method, a word method, or various other methods.

«First Stage Selection Step»

In the present specification, the first stage selection step S104 (Fig. 2), the first step of first stage selection S204 and the second step of first stage selection S214 (Fig. 3), and the first first-stage selection step S304 and the second first-stage selection step S314 (Fig. 4) will be collectively referred to as "first stage selection step" in some cases.

(First Aspect: First Stage Selection Step S104)

This step is shown as "first stage selection" (S104) in Fig. 2.

In the first aspect, the (d) first stage selection step is a step of performing first stage selection of the base sequence of the primer candidate for PCR amplifying the target region based on the local alignment score.

(Second Aspect: First Step of First Stage Selection S204 and Second Step of First Stage Selection S214)

These steps are shown as "first step of first stage selection" (S204) and "second step of first stage selection" (S214) in Fig. 3.

In the second aspect, the ($d_1$) first step of first stage selection is a step of performing first stage selection of the base sequence of the primer candidate for PCR amplifying the first target region based on the local alignment score. The ($d_2$) second step of first stage selection is a step of performing first stage selection of the base sequence of the primer candidate for PCR amplifying the second target region based on the local alignment score.

(Third Aspect: First First-Stage Selection Step S304 and Second First-Stage Selection Step S314)

These steps are shown as "first first-stage selection" (S304) and "second first-stage selection" (S314) in Fig. 4.

In the third aspect, the (d-1) first first-stage selection step is a step of performing first stage selection of the base sequence of the primer candidate for PCR amplifying the first target region based on the local alignment score. The (d-2) second first-stage selection step is a step of performing first stage selection of the base sequence of the primer candidate for PCR amplifying the second target region based on the local alignment score.

(First Stage Selection Method)

A threshold value (also referred to as a first threshold value) of the local alignment score is predetermined.

In a case where a local alignment score is less than the first threshold value, it is determined that the combination of these two base sequences has low dimer formability, and the following step is performed.

In contrast, in a case where a local alignment score is greater than or equal to the first threshold value, it is determined that a combination of these two base sequences has high primer-dimer formability, and the following step is not performed on the combination.

The first threshold value is not particularly limited and can be appropriately set. For example, the first threshold value may be set according to PCR conditions such as an amount of genomic DNA used as a template for a polymerase chain reaction.

Here, in the example shown in the above-described "local alignment step," a case where the first threshold value is set to "+ 3" is considered.

In the above-described example, the local alignment score is "+1" and is less than "+ 3" which is the first threshold value. Therefore, it is possible to determine that the combination of the base sequences of SEQ ID No: 1 and SEQ ID No: 2 has low primer-dimer formability.

The present step is performed on each of combinations which are obtainable with local alignment scores calculated in the local alignment step S103, the first step of local alignment S203, the second step of local alignment S213, the first local alignment step S303, or the second local alignment step S313.

<<Global Alignment Step>>

In the present specification, the global alignment step S105 (Fig. 2), the first step of global alignment S205 and the second step of global alignment S215 (Fig. 3), and the first global alignment step S305 and the second global alignment step S315 (Fig. 4) will be collectively referred to as a "global alignment step" in some cases.

(First Aspect: Global Alignment Step S105)

This step is shown as "global alignment" (S105) in Fig. 2.

In the first aspect, the (e) global alignment step is a step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first stage selection step.

(Second Aspect: First Step of Global Alignment S205 and Second Step of Global Alignment S215)

These steps are shown as "first step of global alignment" (S205) and "second step of global alignment" (S215) in Fig. 3.

In the second aspect, the ($e_1$) first step of global alignment is a step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first step of first stage selection. The ($e_2$) second step of global alignment is a step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed.

(Third Aspect: First Global Alignment Step S305 and Second Global Alignment Step S315)

These steps are shown as "first global alignment" (S305) and "second global alignment" (S315) in Fig. 4.

In the third aspect, the (e-1) first global alignment step is a step of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first first-stage selection step. The (e-2) second global alignment step is a step of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed.

(Global Alignment Method)

The global alignment score is obtained by selecting two primers from the group consisting of all of the primer candidates generated in the "primer candidate base sequence generation step" (in a case where the "local alignment step" and the "first stage selection step" are performed first, and in a case where there are combinations of primer candidates in which a local alignment score is less than the first threshold value, all of the primer candidates included in the combinations thereof), and all of the primers that have already been employed (limited to cases where primers that have already been employed are present); and performing global alignment in a pairwise manner for the base sequence with a predetermined sequence length which includes 3' terminals.

A combination of base sequences to be subjected to global alignment may be a combination selected while allowing overlapping, or may be a combination selected without allowing overlapping. However, in a case where formability of a primer-dimer between primers of an identical base sequence has not yet been evaluated, the combination selected while allowing overlapping is preferable.

The total number of combinations is "$_xH_2 = {}_{x+1}C_2 = (x+1)! / 2(x-1)!$" in a case where the selection is performed while allowing overlapping, and is "$_xC_2 = x(x-1) / 2$" in a case where the selection is performed without allowing overlapping, in which the total number of base sequences for performing global alignment is set to be x.

Global alignment is an alignment which is performed on the entire sequence and in which it is possible to check complementarity of the entire sequence.

However, here, the "entire sequence" refers to the entirety of a base sequence which has a predetermined sequence length and includes the 3' terminal of a base sequence of a primer candidate.

Global alignment may be performed by inserting a gap. The gap means insertion and/or deletion (indel) of a base.

In addition, in the global alignment, a case where bases are complementary to each other between base sequence pairs is regarded as a match and a case where bases are not complementary to each other therebetween is regarded as a mismatch.

Alignment is performed such that scores for each of the match, the mismatch, and the indel are given and the total score becomes a maximum. The score may be appropriately set. For example, scores may be set as in Table 1. "-" in Table 1 represents a gap (insertion and/or deletion (indel)).

For example, it is considered that global alignment is performed on three bases (refer to portions with capital letters) at the 3' terminal of each base sequence of SEQ ID No: 1 and SEQ ID No: 2 shown in the following Table 5. Here, scores are as shown in Table 1.

[Table 5]

| | Base sequence (5'→3') |
|---|---|
| SEQ ID No: 1 | cgctcttccgatctctggcttggccttgggaatgTGG |
| SEQ ID No: 2 | cgctcttccgatctgacggcaatatggccaatgaTGG |

In a case of performing global alignment on base sequences of the three bases (portion with capital letters) at the 3' terminal of the base sequence of SEQ ID No: 1 and the three bases (portion with capital letters) at the 3' terminal of SEQ ID No: 2 such that the score becomes a maximum, it is possible to obtain alignment (pairwise alignment) shown in the following Table 6. In Table 6, mismatches are represented by ":."

[Table 6]

| | |
|---|---|
| 3' Terminal 3 bases of SEQ ID No: 1: | 5'- T G G - 3' |
| 3' Terminal 3 bases of SEQ ID No: 2: | 3'- G G T - 5' |

In this (pairwise) alignment, there are three mismatches, and there is no matches and indels (gap).

Therefore, a global alignment score based on this (pairwise) alignment is $(+ 1) \times 0 + (-1) \times 3 + (- 1) \times 0 = -3$.

The alignment (pairwise alignment) can be obtained through the dot matrix method a dynamic programming method, a word method, or various other methods.

<<Second Stage Selection Step>>

In the present specification, the second stage selection step S106 (Fig. 2), the first step of second stage selection S206 and the second step of second stage selection S216 (Fig. 3), and the first second-stage selection step S306 and the second second-stage selection step S316 will be collectively referred to as a "second stage selection step" in some cases.

(First Aspect: Second Stage Selection Step S106)

This step is shown as "second stage selection" (S106) in Fig. 2.

In the first aspect, the (f) second stage selection step is a step of performing second stage selection of the base sequence of the primer candidate for PCR amplifying the target region based on the global alignment score.

(Second Aspect: First Step of Second Stage Selection S206 and Second Step of Second Stage Selection S216)

These steps are shown as "first step of second stage selection" (S206) and "second step of second stage selection" (S216) in Fig. 3.

In the second aspect, the ($f_1$) first step of second stage selection is a step of performing second stage selection of the base sequence of the primer candidate for PCR amplifying the first target region based on the global alignment score. The ($f_2$) second step of second stage selection is a step of performing second stage selection of the base sequence of the primer candidate for PCR amplifying the second target region based on the global alignment score.

(Third Aspect: First Second-Stage Selection Step S306 and Second Second-Stage Selection Step S316)

These steps are shown as "first second-stage selection" (S306) and "second second-stage selection" (S316) in Fig. 4.

In the third aspect, the (f-1) first second-stage selection step is a step of performing second stage selection of the base sequence of the primer candidate for PCR amplifying the first target region based on the global alignment score. The (f-2) second second-stage selection step is a step of performing second stage selection of the base sequence of the primer candidate for PCR amplifying the second target region based on the global alignment score.

(Second Stage Selection Method)

A threshold value (also referred to as a "second threshold value") of the global alignment score is predetermined.

In a case where a global alignment score is less than the second threshold value, it is determined that a combination of these two base sequences has low dimer formability, and the following step is performed.

In contrast, in a case where a global alignment score is greater than or equal to the second threshold value, it is determined that a combination of these two base sequences has high dimer formability, and the following step is not performed on the combination.

The second threshold value is not particularly limited and can be appropriately set. For example, the second threshold value may be set according to PCR conditions such as an amount of genomic DNA used as a template for a polymerase chain reaction.

It is possible to set the global alignment score obtained by performing pairwise global alignment on a base sequence which has a predetermined number of bases and includes the 3' terminal of a base sequence of each primer to be less than the second threshold value by setting a base sequence with several bases from the 3' terminal of a primer as an identical base sequence.

Here, in the example shown in the above-described "global alignment step," a case where the second threshold value is set to "+ 3" is considered.

In the above-described example, the global alignment score is "-3" and is less than "+3" which is the second threshold value. Therefore, it is possible to determine that the combination of the base sequences of SEQ ID No: 1 and SEQ ID No: 2 has low primer-dimer formability.

The present step is performed on each of combinations which are obtainable with global alignment scores calculated in the global alignment step S105, the first step of global alignment S205, the second step of global alignment S215, the first global alignment step S305, or the second global alignment step S315.

In addition, in order to reduce the amount of calculation, it is preferable to first perform both steps of the "global alignment step" and the "second stage selection step" and to perform both steps of the "local alignment step" and the "first stage selection step" in a combination of the base sequences of the primers, which has passed the "second stage selection step." Particularly, as the number of target regions and the number of base sequences of primer candidates are increased, the effect of reducing the amount of calculation is increased, and it is possible to speed up the overall processing.

This is because the amount of calculation of a global alignment score is smaller than that of a local alignment score which is obtained by searching a partial sequence with high complementarity from the entire base sequence under the condition that the base sequence includes the 3' terminal and it is possible to speed up the processing since global alignment is performed on a base sequence with a short length called a "predetermined sequence length" in the "global alignment step." It is known that the global alignment is faster than the local alignment in a case of alignment with respect to a sequence having an identical length in a well-known algorithm.

«Amplification Sequence Length-Checking Step»

An amplification sequence length-checking step of calculating the distance between ends of base sequences of primer candidates for which it has been determined that formability of a primer-dimer is low in the "first stage selection step" and "second stage selection step" on chromosomal DNA regarding combinations of the base sequences of the primer candidates, and determining whether the distance is within a predetermined range may be performed.

In a case where the distance between the ends of the base sequences is within the predetermined range, it is possible to determine that there is a high possibility that the combinations of the base sequences of the primer candidates can appropriately amplify a target region. The distance between the ends of the base sequences of the primer candidates is not particularly limited, and can be appropriately set in accordance with the PCR condition such as the type of enzyme (DNA polymerase). For example, the distance between the ends of the base sequences of the primer candidates can be set to be within various ranges such as a range of 100 to 200 bases (pair), a range of 120 to 180 bases (pair), a range of 140 to 180 bases (pair) a range of 140 to 160 bases (pair), and a range of 160 to 180 bases (pair).

<<Primer Employment Step>>

In the present specification, the primer employment step S107 (Fig. 2), the first step of primer employment S207 and the second step of primer employment S217 (Fig. 3), and the first primer employment step S307 and the second primer employment step S317 (Fig. 4) will be collectively referred to as a "primer employment step" in some cases.

(First Aspect: Primer Employment Step S107)

This step is shown as "primer employment" (S107) in Fig. 2.

In the first aspect, the (g) primer employment step is a step of employing the base sequence of the primer candidate which is selected in both of the first stage selection step and the second stage selection step as the base sequence of the primer for PCR amplifying the target region.

(Second Aspect: First Step of Primer Employment S207 and Second Step of Primer Employment S217)

These steps are shown as "first step of primer employment" (S207) and "second step of primer employment" (S217) in Fig. 3.

In the second aspect, the ($g_1$) first step of primer employment is a step of employing the base sequence of the primer candidate which is selected in both of the first step of first stage selection and the first step of second stage selection as a base sequence of a primer for PCR amplifying the first target region. The ($g_2$) second step of primer employment is a step of employing the base sequence of the primer candidate which is selected in both of the second step of first stage selection and the second step of second stage selection as a base sequence of a primer for PCR amplifying the second target region.

(Third Aspect: First Primer Employment Step S307 and Second Primer Employment Step S317)

These steps are shown as "first primer employment" (S307) and "second primer employment" (S317) in Fig. 4.

In the third aspect, the (g-1) first primer employment step is a step of employing the base sequence of the primer candidate which is selected in both of the first first-stage selection step and the first second-stage selection step as the base sequence of the primer for PCR amplifying the first target region. The (g-2) second primer employment step is a step of employing the base sequence of the primer candidate which is selected in both of the second first-stage selection step and the second second-stage selection step as the base sequence of the primer for PCR amplifying the second target region.

(Primer Employment Method)

In the primer employment step, the base sequence of the primer candidate, in which a local alignment score obtained by performing pairwise local alignment on a base sequence of each primer candidate under a condition that a partial sequence to be subjected to comparison includes the 3' terminal of the base sequence is less than the first threshold value, and a global alignment score obtained by performing pairwise global alignment on a base sequence which has a predetermined number of bases and includes the 3' terminal of the base sequence of each primer candidate is less than the second threshold value, is employed as the base sequence of the primer for amplifying a target region.

For example, it is considered that base sequences of SEQ ID No: 1 and SEQ ID No: 2 shown in Table 7 are employed as base sequences of primers for amplifying a target region.

[Table 7]

| | Base sequence (5'→3') |
|---|---|
| SEQ ID No: 1: | CGCTCTTCCGATCTCTGGCTTGGCCTTGGGAATGTGG |
| SEQ ID No: 2: | CGCTCTTCCGATCTGACGGCAATATGGCCAATGATGG |

As already described, the local alignment score is "+1" and thus is less than "+3" which is the first threshold value, and the global alignment score is "-3" and thus is less than "+3" which is the second threshold value.
Accordingly, it is possible to employ the base sequence of the primer candidate represented by SEQ ID No: 1 and the base sequence of primer candidate represented by SEQ ID No: 2 as base sequences of primers for amplifying a target region.

<<Primer Design of Other Objective Regions>>

After employing a primer for one objective region, primers for another objective region may further be designed.
In the first aspect, in the primer candidate base sequence generation step S102, in a case where a base sequence of a primer candidate for an objective region for which a primer is to be designed is already generated, steps are carried out from the local alignment step S103. In a case where a base sequence of a primer candidate for the next objective region is not yet generated, because the next objective region is not selected in the target region selection step S101, the next objective region is selected in the target region selection step S101, and a base sequence of a primer candidate for the objective region is generated in the primer candidate base sequence generation step S102, and then steps subsequent to the local alignment step S103 are carried out.
In the second aspect, steps are repeated from the second step of target region selection S211.
In the third aspect, since the base sequence of the primer candidate for the objective region already selected in the target region multiple selection step S301 is already generated in the primer candidate base sequence multiple generation step S302, steps are repeated from the second local alignment step S313.

<<Characteristic Points in Design of Primers and the Like>>

Characteristic points in the design of the primers and the like after selecting the objective region in general are as follows: obtaining primer groups which include target regions as targets to be amplified and do not become complementary to each other, by selecting a plurality of specific target regions, searching vicinity base sequences, checking complementarity with each extracted primer set, and selecting base sequences having low complementarity.
Characteristic points in checking of the complementarity of a base sequence of a primer are that primer groups are generated such that complementarity of a whole sequence becomes low using local alignment and the complementarity with respect to ends of a base sequence of a primer becomes low using global alignment.
Hereinafter, the present invention will be described in more detail using Examples, but is not limited to these Examples.

Examples

[Example 1]

<Acquisition of Base Sequence Information of Single Cell of Nucleated Red Blood Cell Derived from Fetus>

<<Selection of Objective Region>>

SNPs on chromosome 13, chromosome 18, chromosome 21, and X chromosome, and Y chromosome-specific regions shown in Table 8 were selected as the objective regions to be amplified by PCR.

[Table 8]

| No. | Amplification target region | | | | | | |
|---|---|---|---|---|---|---|---|
| | Gene name | refSNP | Chromosome | SNP coordinate | Allyl | Start coordinate | End coordinate |
| 1 | ATP12A | rs7981616 | 13 | 25265103 | A/G | 25264999 | 25265178 |
| 2 | KIAA0226L | rs1408184 | 13 | 46946157 | C/T | 46946120 | 46946292 |
| 3 | ATP7B | rs1801244 | 13 | 52544805 | C/G | 52544717 | 52544880 |
| 4 | DOCK9 | rs2296984 | 13 | 99457431 | T/G | 99457303 | 99457464 |
| 5 | TMTC4 | rs946837 | 13 | 101287340 | C/G, T | 101287239 | 101287398 |
| 6 | DSG2 | rs2230233 | 18 | 29104698 | C/T | 29104659 | 29104819 |

(continued)

| No. | Amplification target region | | | | | | |
|---|---|---|---|---|---|---|---|
| | Gene name | refSNP | Chromosome | SNP coordinate | Allyl | Start coordinate | End coordinate |
| 7 | KATNAL2 | rs7233515 | 18 | 44585955 | G/A, T | 44585836 | 44585977 |
| 8 | MRO | rs4940019 | 18 | 48333203 | C/G | 48333122 | 48333301 |
| 9 | ALPK2 | rs3809971 | 18 | 56204977 | C/T | 56204947 | 56205121 |
| 10 | ADNP2 | rs3744877 | 18 | 77894844 | G/A | 77894713 | 77894884 |
| 11 | ITSN1 | rs2073370 | 21 | 35260481 | T/C | 35260401 | 35260571 |
| 12 | BRWD1 | rs1041439 | 21 | 40571246 | A/G | 40571215 | 40571388 |
| 13 | B3GALT5 | rs3746887 | 21 | 41032740 | T/C | 41032678 | 41032844 |
| 14 | UMODL1 | rs220312 | 21 | 43519032 | G/A | 43518893 | 43519063 |
| 15 | NDUFV3 | rs4148973 | 21 | 44323590 | T/G | 44323536 | 44323703 |
| 16 | PUDP | rs2379206 | X | 6995315 | C/T | 6995304 | 6995473 |
| 17 | CLDN34 | rs5934730 | X | 9935526 | G/T | 9935444 | 9935607 |
| 18 | MAGEB1 | rs2071311 | X | 30261002 | A/G | 30260896 | 30261067 |
| 19 | DMD | rs1801187 | X | 32380996 | C/T | 32380928 | 32381102 |
| 20 | TBC1D25 | rs2293948 | X | 48418126 | A/G | 48418093 | 48418271 |
| 21 | | | | | | 3609387 | 3609547 |
| 22 | | | Y | | | 7697094 | 7697262 |
| 23 | | | Y | | | 18206326 | 18206492 |
| 24 | | | Y | | | 23424722 | 23424894 |
| 25 | | | Y | | | 6976908 | 6977061 |

<<Single Cell Isolation>>

(Acquisition of Peripheral Blood Sample)

10.5 mg of sodium salts of ethylenediaminetetraacetic acid (EDTA) was added to a 7 mL blood collecting tube as an anticoagulant, and then, 7 mL of peripheral blood was obtained within the blood collecting tube as volunteer blood after obtaining informed consent from a pregnant woman volunteer. Thereafter, the blood was diluted using physiological salt solution.

(Concentration of Nucleated Red Blood Cell)

Liquids with a density of 1.070 and a density of 1.095 were prepared using PERCOLL LIQUID (registered trademark; manufactured by Sigma-Aldrich Co.), 2 mL of the liquid with a density of 1.095 was added to the bottom portion of a centrifuge tube, and the centrifuge tube was cooled in a refrigerator for 30 minutes at 4°C. Thereafter, the centrifuge tube was taken out from the refrigerator and 2 mL of a liquid with a density of 1.070 was made to slowly overlap the top of the liquid with a density of 1.095 so as not to disturb the interface. Then, 11 mL of diluent of blood which had been collected above was slowly added to the top of the medium with a density of 1.070 in the centrifuge tube. Thereafter, centrifugation was performed for 20 minutes at 2000 rpm. The centrifuge tube was taken out, and the fraction deposited between the liquid of the density 1.070 and the liquid of the density 1.095 was collected using a pipette. The blood fraction thus collected was washed twice with a washing solution (2 mM ethylenediaminetetraacetic acid (EDTA) and 0.1% bovine serum albumin (BSA)-phosphate buffered saline (PBS)), and then nucleated red blood cell fraction was obtained.

(Detection and Isolation of Nucleated Red Blood Cell)

The number of cells in the nucleated red blood cell fraction was calculated with a cell counter, and was adjusted with fluorescence activated cell sorting (FACS) buffer solution (0.1% bovine serum albumin (BSA)-phosphate buffered saline (PBS)) so that a cell concentration became $1 \times 10^7$ cells/mL. Thereafter, 10 μL of BV421-labeled anti-CD45 antibody (manufactured by BD Bioscience) as a white blood cell marker to dye nucleated red blood cells in different colors, 15 μL of fluorescein isothiocyanate (FITC) labeled anti-CD71 antibody (manufactured by BD Bioscience) as a juvenile marker of red blood cells, and 1 μL of DRAQ 5 (manufactured by Cell Signaling Technology Co., Ltd.) as nuclear staining were added and incubated at 4°C for 30 minutes or longer.

The cell suspension after staining was subjected to a flow cytometer (SH800ZP manufactured by SONY CORPORATION) to perform gate setting of CD45-negative, CD71-positive, and DRAQ5-positive fractions as nucleated red blood cell fractions, and single cell sorting was carried out in a yield mode. In order to check the number and types of sorted cells, the nucleated red blood cell fractions were sorted one by one into 96-well plates (manufactured by Gravity Trap) capable of capturing an image and picking up cells, and only wells containing one nucleated red blood cell were isolated in a PCR tube using the captured image.

<<Extraction of Genomic DNA>>

(Cell Lysis)

Isolated single cells were lysed in the PCR tube. As reaction conditions, each cell was added with 5 μL of a cell lysis buffer solution (10 mM Tris-Hcl, pH 7.5, 0.5 mM EDTA, 20 mM KCl, 0.007% (w/v), sodium dodecyl sulfate (SDS), 13.3 μg/mL proteinase K).

Each PCR tube was heated at 50°C for 60 minutes, and protein was dissolved with Proteinase K. Next, each tube was heated at 95°C for 5 minutes to inactivate Proteinase K.

Accordingly, genomic DNA was prepared.

<<Preparation of Primer>>

In order to analyze fetal cells and analyze numerical abnormality of chromosomes, a primer set to be subjected to multiplex PCR was prepared from the base sequence near a target region to be amplified shown in Table 8.

The primer that has less than "+3" of a local alignment score of 3' terminal fixed between each primer and has less than "0" of a global alignment score of 3' terminal 3 bases of each primer so that a Tm value became 56°C to 64°C and a PCR amplification base length became 140 to 180 base pairs, was selected, and 27 pairs of primer pairs were prepared.

(Confirmation of Amplification through Singleplex PCR)

In order to confirm that the prepared primer pair amplifies the target region to be amplified, the reaction was carried out using the Multiplex PCR Assay kit (manufactured by Takara Bio Inc.).

a) Preparation of reaction liquid

The following reaction liquid was prepared.

| | |
|---|---|
| Genomic DNA (0.5 ng/μL) | 2 μL |
| Primers | 2 μL for each |
| Multiplex PCR Mix 1 | 0.125 μL |
| Multiplex PCR Mix 2 | 12.5 μL |
| Sterilized distilled water | up to 25 μL |

The genomic DNA is genomic DNA extracted from human cultured cells, and Multiplex PCR Mix 1 and Multiplex PCR Mix 2 are reagents contained in TaKaRa Multiplex PCR Assay Kit (manufactured by Takara Bio Inc.).

b) Reaction conditions for singleplex PCR

As reaction conditions for singleplex PCR, 30 cycles of thermal cycle: [thermal denaturation (94°C, 30 seconds) - annealing (60°C, 90 seconds) - extension (72°C, 30 seconds)] were carried out after initial thermal denaturation (94°C, 60 seconds).

c) Confirmation of PCR Amplification Product

A portion of the reaction liquid after completion of the reaction was subjected to agarose gel electrophoresis, and the presence or absence and size of the PCR amplification product were confirmed.

Specific amplification by singleplex PCR could be confirmed for 25 pairs of primers in Table 9.

[Table 9]

| No. | Primer | | | | Amplification product |
|---|---|---|---|---|---|
| | Name | Base sequence (5' →3') | Size (mer) | SEQ ID NO | Size (bp) |
| 1 | 1F | cgctcttccgatctctgGCTTGGCCTTGGGAATGTGG | 37 | 1 | 180 |
| | 1R | cgctcttccgatctgacGGCAATATGGCCAATGATGG | 37 | 2 | |
| 2 | 2F | cgctcttccgatctctgCTGTCAGTCTCAGGATATGG | 37 | 3 | 173 |
| | 2R | cgctcttccgatctgacGATACCACAGACTCCGTTGG | 37 | 4 | |
| 3 | 3F | cgctcttccgatctctgACTGCTCTGGTTGATTGTGG | 37 | 5 | 164 |
| | 3R | cgctcttccgatctgacTGTTCTACTAACCCTCTTGG | 37 | 6 | |
| 4 | 4F | cgctcttccgatctctgTTCCCGGTCTGCGTAAATGG | 37 | 7 | 162 |
| | 4R | cgctcttccgatctgacGGTCAACCCTAAGGATCTGG | 37 | 8 | |
| 5 | 5F | cgctcttccgatctctgTCATTCTGTTCATCAGCTGG | 37 | 9 | 160 |
| | 5R | cgctcttccgatctgacTAACCTGTTCTTCCGAGTGG | 37 | 10 | |
| 6 | 6F | cgctcttccgatctctgTTTGCAGCTTGAAGGGATGG | 37 | 11 | 161 |
| | 6R | cgctcttccgatctgacGAGCATCTGTTTCTATGTGG | 37 | 12 | |
| 7 | 7F | cgctcttccgatctctgCATCGGACTTTGCTTGATGG | 37 | 13 | 142 |
| | 7R | cgctcttccgatctgacTATATGTAGGCCGAAGTTGG | 37 | 14 | |
| 8 | 8F | cgctcttccgatctctgGTGACGCTTTTTAGCACTGG | 37 | 15 | 180 |
| | 8R | cgctcttccgatctgacTCTTTAGAGGGAGAGATTGG | 37 | 16 | |
| 9 | 9F | cgctcttccgatctctgCCCAACAAGAGAATCTATGG | 37 | 17 | 17 5 |
| | 9R | cgctcttccgatctgacTGACTTCAGGGAGCCTGTGG | 37 | 18 | |
| 10 | 10F | cgctcttccgatctctgTCTGGGGTTCTTCCTACTGG | 37 | 19 | 172 |
| | 10R | cgctcttccgatctgacCTGAGGAGGAGACTGTCTGG | 37 | 20 | |
| 11 | 11F | cgctcttccgatctctgGCCTCGAAGAGAGGGAATGG | 37 | 21 | 171 |
| | 11R | cgctcttccgatctgacGACCACAATCTCTCCCGTGG | 37 | 22 | |
| 12 | 12F | cgctcttccgatctctgCTGGGCAGTGTGAGAACTGG | 37 | 23 | 174 |
| | 12R | cgctcttccgatctgacTCTGAAAGTGTCTGTTCTGG | 37 | 24 | |
| 13 | 13F | cgctcttccgatctctgCTCATCCCACAAACAGTTGG | 37 | 25 | 167 |
| | 13R | cgctcttccgatctgacTAATGTCCCCGTGTCGCTGG | 37 | 26 | |
| 14 | 14F | cgctcttccgatctctgAATAGCCAGTGCTGTTCTGG | 37 | 27 | 171 |
| | 14R | cgctcttccgatctgacACCACGTAGTCACTGACTGG | 37 | 28 | |

(continued)

| No. | Primer | | | | Amplification product |
|---|---|---|---|---|---|
| | Name | Base sequence (5' →3') | Size (mer) | SEQ ID NO | Size (bp) |
| 15 | 15F | cgctcttccgatctctgTTCAGAAGCTCGTCAGGTGG | 37 | 29 | 168 |
| | 15R | cgctcttccgatctgacAAGGAATGAGAGGCCTCTGG | 37 | 30 | |
| 16 | 16F | cgctcttccgatctctgAGGAAGATGTCCGGGTCTGG | 37 | 31 | 170 |
| | 16R | cgctcttccgatctgacATCCACCTGCGGAAACATGG | 37 | 32 | |
| 17 | 17F | cgctcttccgatctctgCCCTTACCACCATAGGATGG | 37 | 33 | 164 |
| | 17R | cgctcttccgatctgacTTTGGTTGTGGTGCTGTTGG | 37 | 34 | |
| 18 | 18F | cgctcttccgatctctgCCCGTGAAGAGGAAATCTGG | 37 | 35 | 172 |
| | 18R | cgctcttccgatctgacCACAGGAATTGATAGCGTGG | 37 | 36 | |
| 19 | 19F | cgctcttccgatctctgAAATGGCTGCAAATCGATGG | 37 | 37 | 175 |
| | 19R | cgctcttccgatctgacGTCCTATCTCTTGCTCATGG | 37 | 38 | |
| 20 | 20F | cgctcttccgatctctgAGCAGCTCAAGAGCGAGTGG | 37 | 39 | 179 |
| | 20R | cgctcttccgatctgacGTGGGTAACGGCATAGGTGG | 37 | 40 | |
| 21 | 21F | cgctcttccgatctctgATACCAGTTACTGGCAATGG | 37 | 41 | 161 |
| | 21R | cgctcttccgatctgacACACAGACAGCGAAAGATGG | 37 | 42 | |
| 22 | 22F | cgctcttccgatctctgGGCAGGTGTCAGGCTTATGG | 37 | 43 | 169 |
| | 22R | cgctcttccgatctgacTGGTGGCCTGGTAAAAGTGG | 37 | 44 | |
| 23 | 23F | cgctcttccgatctctgCAGACCGAAACAAGGGTTGG | 37 | 45 | 167 |
| | 23R | cgctcttccgatctgacCTTGGAAGGTATAGCTCTGG | 37 | 46 | |
| 24 | 24F | cgctcttccgatctctgGGCTGAATTCTTGTGACTGG | 37 | 47 | 173 |
| | 24R | cgctcttccgatctgacTCCCACAACACTGAGCATGG | 37 | 48 | |
| 25 | 25F | cgctcttccgatctctgTGTTATGCTTGGGTGAATGG | 37 | 49 | 154 |
| | 25R | cgctcttccgatctgacTACAGTGAGAGAGAGCTTGG | 37 | 50 | |

<<Amplification of Objective Region>>

(Amplification by Multiplex PCR)

a) Preparation of reaction liquid

The following reaction liquid was prepared.

| | |
|---|---|
| Template DNA | 9 μL |
| Primer · mix | 4 μL |
| Multiplex PCR Mix 1 | 0.125 μL |
| Multiplex PCR Mix 2 | 12.5 μL |
| Sterilized distilled water | up to 26 μL |

Template DNA is genomic DNA extracted from a single cell, and primer mix is obtained by mixing 25 pairs of primer pairs in which amplification with singleplex could be confirmed so that a final concentration of each primer became 50 nM, and Multiplex PCR Mix 1 and Multiplex PCR Mix 2 are reagents contained in TaKaRa Multiplex PCR Assay Kit (manufactured by Takara Bio Inc.).

b) Reaction conditions for Multiplex PCR

As reaction conditions for Multiplex PCR, 32 cycles of thermal cycle: [thermal denaturation (94°C, 30 seconds) - annealing (56.7°C, 600 seconds) - extension (72°C, 30 seconds)] were carried out after initial thermal denaturation (94°C, 60 seconds).

c) Purification of PCR Reaction Product

The PCR reaction product obtained by multiplex PCR was purified according to the attached manual (Instructions For Use) using Agencourt AMPure XP (Beckman Coulter).

Specifically, 45 μL of the AMPure XP reagent was added to 25 μL of the PCR reaction liquid, mixed thoroughly, and allowed to stand at room temperature for 5 minutes to bind the PCR reaction product to magnetic beads.

Next, the magnetic beads to which the PCR reaction product was bound were separated by magnetic force of a magnet stand (MagnaStand, manufactured by Japan Genetics Co., Ltd.) to remove contaminants.

After washing the magnetic beads to which the PCR reaction product was bound twice with 70% (v/v) ethanol, DNA bound to the magnetic beads was eluted with 40 μL of TE (Tris-EDTA) buffer solution.

<<DNA Sequencing>>

(Preparation of Sequence Library Mix)

In order to perform dual index sequencing using a next generation sequencer (Miseq, Illumina Co.), two kinds of adapters including a flow cell binding sequence (P5 sequence or P7 sequence), an index sequence for sample identification, and a sequence primer binding sequence were added to both ends of a DNA fragment obtained by multiplex PCR, respectively.

Specifically, addition of sequences was performed to both ends by performing PCR using the Multiplex PCR Assay kit and using 1.25 μM of each of primers D501-F and D701-R to D706-R (shown in Table 10). As PCR conditions, 5 cycles of thermal cycle: [thermal denaturation (94°C, 45 seconds) - annealing (50°C, 60 seconds) - extension (72°C, 30 seconds)] were carried out after initial thermal denaturation (94°C, 3 minutes), and then 11 cycles of thermal cycle: [thermal denaturation (94°C, 45 seconds) - annealing (55°C, 60 seconds) - extension (72°C, 30 seconds)] were carried out.

[Table 10]

| Primer | | | |
|---|---|---|---|
| Name | Base sequence (5' →3') | Size (mer) | SEQ ID NO |
| D501-F | AATGATACGGCGACCACCGAGATCTACACtatagcctTCTTTCCCTACACGACGCTCTTCCGATCTCTG | 69 | 51 |
| D701-R | CAAGCAGAAGACGGCATACGAGATcgagtaatGTGACTGGAGTTCAGACGTGTGCTCTTCCGATCTGAC | 69 | 52 |
| D702-R | CAAGCAGAAGACGGCATACGAGATtctccggaGTGACTGGAGTTCAGACGTGTGTCTTCCGATCTGAC | 69 | 53 |
| D703-R | CAAGCAGAAGACGGCATACGAGATaatgagcgGTGACTGGAGTTCAGACGTGTGCTCTTCCGATCTGAC | 69 | 54 |
| D704-R | CAAGCAGAAGACGGCATACGAGATggaatctcGTGACTGGAGTTCAGACGTGTGCTCTTCCGATCTGAC | 69 | 55 |
| D705-R | CAAGCAGAAGACGGCATACGAGATttctgaatGTGACTGGAGTTCAGACGTGTGCTCTTCCGATCTGAC | 69 | 56 |
| D706-R | CAAGCAGAAGACGGCATACGAGATacgaattcGTGACTGGAGTTCAGACGTGTGCTCTTCCGATCTGAC | 69 | 57 |

The obtained PCR product was purified using Agencourt AMPure XP (Beckman Coulter, Inc.), and the DNA was quantitatively determined using KAPA Library Quantification Kits (manufactured by Japan Genetics Co., Ltd.).
Each sample with different index sequences was mixed so that a DNA concentration became 1.5 pM, and used as a sequence library mix.

(Sequence Analysis of Amplification Product)

The prepared sequence library mix was sequenced using Miseq Reagent Kit v2 300 Cycle (manufactured by Illumina), and therefore a FastQ file was obtained.
After mapping a human genome sequence (hg19) from the obtained FastQ data using BWA (Burrows-Wheeler Aligner), gene polymorphism information was extracted by SAMtools, and the number of sequence reading of each detection region was calculated by BEDtools.

(Confirmation of Cell Information)

It was possible to confirm that 4 cells out of 24 cells had different types of SNP information by comparing SNPs of chromosome 13, chromosome 18, and chromosome 21 of each of the PCR amplification products amplified from each cell.
Separately, genomic DNA was extracted from the nucleated cell fraction after being Percoll-concentrated with QIAamp DNA Mini Kit (Qiagen Co., Ltd.) in order to obtain maternal SNP information. DNA was amplified in the same manner using 10 ng of genomic DNA as a template, and when the SNP was examined, it was confirmed that DNA matched with the SNP of 20 cells. From the above, it was confirmed that 4 cells were defined as a fetus-derived nucleated red blood cell and 20 cells were defined as mother-derived nucleated cells.
The results are shown in Fig. 5. 20 cells other than cells 3, 8, 18, and 20 show the same SNP pattern as that of the sample genome (the genome extracted from nucleated cells derived from the mother), which teaches that these 20 cells derived from the mother. Cell 3, cell 8, cell 18, and cell 20 were presumed to be derived from a fetus.

(Detection of Amount of Amplification Product)

The amount of amplification product of a detection region of chromosome 18 of a nucleated red blood cell which was identified as being derived from a fetus and was discriminated through sequence analysis of a PCR amplification product was defined through sequencing the amplification product using Miseq (registered trademark, manufactured by Illumina, Inc.) which was a next generation sequencer.
Separately, the amount of amplification products (number of times of sequence reading) of detection regions of chromosome 18 of nucleated cells which were identified as being derived from a mother were confirmed by performing sequencing of the amplification products using Miseq.
The variation between cells was small as a result of comparing the amounts of these amplification products, and the proportions of the amounts of these two amplification products were calculated. As a result, it was assumed that the proportions were values close to 1:1.5 and fetus was with trisomy.
When an amount ratio of the amplification product of the mother cell and the nucleated cell identified was calculated in the same manner with respect to chromosome 13 and chromosome 21, both of chromosome 13 and chromosome 21 had values close to 1:1.
Fig. 6 shows these results. The number of chromosome 18 of cells 3, 8, 18, and 20 was about 1.5 times the number of each of chromosome 13 and chromosome 21, and therefore chromosome 18 trisomy of the fetus was assumed.

[Comparative Example 1]

In the multiplex PCR amplification step, primer pairs in which complementarity between primers is not considered were designed instead of primer pairs which were used in Example 1 and designed such that complementarity between primers is reduced, for comparison for checking the effect of calculating the complementarity. In regard to designing of primers, with respect to the same chromosomal location or gene as that of Example 1, each of 20 bp primers of which a Tm value was 56 to 64 and a PCR amplification base length was 140 to 180 base pairs was prepared using Primer 3.
Sequence determination of the amplification products was performed in the same manner as in Example 1 except that multiplex PCR was performed using these primer pairs. As a result, a mapping rate was significantly decreased and distribution of the amount of amplification product between cells was large. The variation between the cells was large enough that numerical abnormality could not be determined clearly.

[Example 2]

<Acquisition of Base Sequence Information of Single Cell of Cancer Cell>

<<Selection of Objective Region>>

A mutation site of a cancer-related gene was selected.

<<Single Cell Isolation>>

Using a cell line H1581 derived from a non-small cell lung cancer, the number of cells was calculated with a cell counter and adjusted with PBS (-) (phosphate buffered saline not containing magnesium and calcium) so that a cell concentration became $1 \times 10^6$ cells/mL. Thereafter, in order to distinguish impurities such as dead cells, the nucleus was stained by adding a cell membrane permeable near infrared fluorescent DNA staining reagent DRAQ5 (manufactured by Abcam Corporation).

The cell suspension after nuclear staining was subjected to a flow cytometer (FACS Aria III, manufactured by Becton, Dickinson and Company), gate setting as a DRAQ5-positive fraction as a cancer cell fraction was performed, and a single cell was sorted and collected in a PCR tube.

<<Extraction of Genomic DNA>>

(Cell Lysis)

Isolated single cells were lysed in the PCR tube. In addition, in the samples recovered with 10 cells and 100 cells by a flow cytometer in the same manner, cell lysis was carried out in the PCR tube. As reaction conditions, each cell was added with 5 μL of a cell lysis buffer solution (10 mM Tris-Hcl, pH 7.5, 0.5 mM EDTA, 20 mM KCl, 0.007% (w/v), sodium dodecyl sulfate (SDS), 13.3 μg/mL proteinase K).

Each PCR tube was heated at 50°C for 60 minutes, and protein was dissolved with Proteinase K. Next, each tube was heated at 95°C for 5 minutes to inactivate Proteinase K.

Accordingly, genomic DNA was prepared.

<<Preparation of Primer>>

For purpose of analyzing a cancer-related gene mutation in cancer cells, primers used for multiplex PCR were prepared from 27 cancer-related gene regions.

The primer that has less than "+3" of a local alignment score of 3' terminal fixed between each primer and has "less than 0" of a global alignment score of 3' terminal 3 bases of each primer so that a Tm value became 56°C to 64°C and a PCR amplification base length became 138 to 300 base pairs, was selected, and 20 pairs of primer pairs were generated.

(Confirmation of Amplification through Singleplex PCR)

In order to confirm that the prepared primer pair amplifies the target region to be amplified, the reaction was carried out using the Multiplex PCR Assay kit (manufactured by Takara Bio Inc.).

a) Preparation of reaction liquid

The following reaction liquid was prepared.

| | |
|---|---|
| Genomic DNA (0.5 ng/μL) | 2 μL |
| Primers | 2 μL for each |
| Multiplex PCR Mix 1 | 0.125 μL |
| Multiplex PCR Mix 2 | 12.5 μL |
| Sterilized distilled water | up to 25 μL |

The genomic DNA is genomic DNA extracted from human cultured cells, and Multiplex PCR Mix 1 and Multiplex PCR Mix 2 are reagents contained in TaKaRa Multiplex PCR Assay Kit (manufactured by Takara Bio Inc.).

b) Reaction conditions for singleplex PCR

As reaction conditions for singleplex PCR, 30 cycles of thermal cycle: [thermal denaturation (94°C, 30 seconds) - annealing (60°C, 90 seconds) - extension (72°C, 30 seconds)] were carried out after initial thermal denaturation (94°C, 60 seconds).

c) Confirmation of PCR Amplification Product

A portion of the reaction liquid after completion of the reaction was subjected to agarose gel electrophoresis, and the presence or absence and size of the PCR amplification product were confirmed.
Specific amplification by singleplex PCR could be confirmed for 20 pairs of primers in Table 11.

[Table 11]

| No. | Primer | | | |
|---|---|---|---|---|
| | Name | Base sequence (5' →3') | Size (mer) | SEQ ID NO |
| 1 | ALK_1F | cgctcttccgatctctgGGTGTCAATTACTGCAGGAAGTGTG | 42 | 58 |
| | ALK_1R | cgctcttccgatctgacGCTGCCAGAAACTGCCTCTTG | 38 | 59 |
| 2 | ALK_2F | cgctcttccgatctctgCACAACAACTGCAGCAAAGACTGG | 41 | 60 |
| | ALK_2R | cgctcttccgatctgacTGTAGCTGCTGAAAATGTAACTTTG | 42 | 61 |
| 3 | ALK_3F | cgctcttccgatctctgGGTTGTTCCATTCTGGTAAGAAGTG | 42 | 62 |
| | ALK_3R | cgctcttccgatctgacGGTAAGAAGTGGCTCACTCTTG | 39 | 63 |
| 4 | FGFR3_1F | cgctcttccgatctctgACAAAAACATCATCAACCTGCTGG | 41 | 64 |
| | FGFR3_1R | cgctcttccgatctgacAGGACACCAGGTCCTTGAAGGTG | 40 | 65 |
| 5 | FGFR3_2F | cgctcttccgatctctgGAGTACTTGGCCTCCCAGAAGGTG | 41 | 66 |
| | FGFR3_2R | cgctcttccgatctgacGTGTGGGAAGGCGGTGTTG | 36 | 67 |
| 6 | FGFR3_3F | cgctcttccgatctctgAGAGCTCAGGCTTCAGGGGTG | 38 | 68 |
| | FGFR3_3R | cgctcttccgatctgacTCACAGGTCGTGTGTGCAGTTG | 39 | 69 |
| 7 | PDGFRA_1F | cgctcttccgatctctgGCATAGCAACCTAGTTCAGTGCTTG | 42 | 70 |
| | PDGFRA_1R | cgctcttccgatctgacTTACCAAGCACTAGTCCATCTCTTG | 42 | 71 |
| 8 | PDGFRA_2F | cgctcttccgatctctgCCTTGAATTGATGGAAGCTCATTGG | 42 | 72 |
| | PDGFRA_2R | cgctcttccgatctgacCCAAAGATATCCAGCTCTTTCTTTG | 42 | 73 |
| 9 | PDGFRA_3F | cgctcttccgatctctgCTTCTCTAGAGCTTTCTCTCTGTTG | 42 | 74 |
| | PDGFRA_3R | cgctcttccgatctgacAGCCTGACCAGTGAGGGAAGTG | 39 | 75 |
| 10 | KIT_1F | cgctcttccgatctctgACCCTGTTCACTCCTTTGCTGATTG | 42 | 76 |
| | KIT_1R | cgctcttccgatctgacCCCATTTGTGATCATAAGGAAGTTG | 42 | 77 |
| 11 | KIT_2F | cgctcttccgatctctgAGAAATTCAGGTTAAAAGAGGCTTG | 42 | 78 |
| | KIT_2R | cgctcttccgatctgacCTTCTGCATGATCTTCCTGCTTTG | 41 | 79 |
| 12 | KIT_3F | cgctcttccgatctctgGAACATCATTCAAGGCGTACTTTTG | 42 | 80 |
| | KIT_3R | cgctcttccgatctgacCAGGACTGTCAAGCAGAGAATGG | 40 | 81 |
| 13 | FGFR4_1F | cgctcttccgatctctgCATGCTCCCTCGTGCAGTTG | 37 | 82 |
| | FGFR4_1R | cgctcttccgatctgacACTCCCGCAGGTTTCCCTTG | 37 | 83 |

(continued)

| No. | Primer | | | |
|---|---|---|---|---|
| | Name | Base sequence (5' →3') | Size (mer) | SEQ ID NO |
| 14 | FGFR4_2F | cgctcttccgatctctgCACGGGCCGTTAGGGTG | 34 | 84 |
| | FGFR4_2R | cgctcttccgatctgacAATGCCACAGGCCTGAGAGTG | 38 | 85 |
| 15 | EGFR_1F | cgctcttccgatctctgAAACGTCCCTGTGCTAGGTCTTTTG | 42 | 86 |
| | EGFR_1R | cgctcttccgatctgacGTACTGGGAGCCAATATTGTCTTTG | 42 | 87 |
| 16 | EGFR_2F | cgctcttccgatctctgTCTGTTTCAGGGCATGAACTACTTG | 42 | 88 |
| | EGFR_2R | cgctcttccgatctgacGACCTAAAGCCACCTCCTTACTTTG | 42 | 89 |
| 17 | MET_1F | cgctcttccgatctctgGCATTTTTATTCAAGAATTCTGTTG | 42 | 90 |
| | MET_1R | cgctcttccgatctgacTACCACATCTGACTTGGTGG | 37 | 91 |
| 18 | FGFRI_1F | cgctcttccgatctctgAGATGATAAGTCACAGGCTGG | 38 | 92 |
| | FGFR1_1R | cgctcttccgatctgacGGTGGGGTTTCTTTGAGGTG | 37 | 93 |
| 19 | FGFR2_1F | cgctcttccgatctctgATAGGAGTACTCCATCCCGGGTG | 40 | 94 |
| | FGFR2_1R | cgctcttccgatctgacTAGAGACGAGGTTTCGCTATGCTGG | 42 | 95 |
| 20 | FGFR2_2F | cgctcttccgatctctgGAACTTTAAACATGCACACAGGGTG | 42 | 96 |
| | FGFR2_2R | cgctcttccgatctgacCATGCTGTTTCAACTAAGTCTTTGG | 42 | 97 |

<<Amplification of Objective Region>>

(Amplification by Multiplex PCR)

a) Preparation of reaction liquid

The following reaction liquid was prepared.

| | |
|---|---|
| Template DNA | 9 μL |
| Primer mix | 4 μL |
| Multiplex PCR Mix 1 | 0.125 μL |
| Multiplex PCR Mix 2 | 12.5 μL |
| Sterilized distilled water | up to 26 μL |

Template DNA is genomic DNA extracted from a single cell isolated from the cancer cells, and primer mix is obtained by mixing 20 pairs of primer pairs in which amplification with singleplex could be confirmed so that a final concentration of each primer became 50 nM, and Multiplex PCR Mix 1 and Multiplex PCR Mix 2 are reagents contained in TaKaRa Multiplex PCR Assay Kit (manufactured by Takara Bio Inc.).

b) Reaction conditions for Multiplex PCR

As reaction conditions for Multiplex PCR, 32 cycles of thermal cycle: [thermal denaturation (94°C, 30 seconds) - annealing (56.7°C, 600 seconds) - extension (72°C, 30 seconds)] were carried out after initial thermal denaturation (94°C, 60 seconds).

c) Purification of PCR Reaction Product

The PCR reaction product obtained by multiplex PCR was purified according to the attached manual (Instructions For Use) using Agencourt AMPure XP (Beckman Coulter).
Specifically, 45 μL of the AMPure XP reagent was added to 25 μL of the PCR reaction liquid, mixed thoroughly, and allowed to stand at room temperature for 5 minutes to bind the PCR reaction product to magnetic beads.

Next, the magnetic beads to which the PCR reaction product was bound were separated by magnetic force of a magnet stand (MagnaStand, manufactured by Japan Genetics Co., Ltd.) to remove contaminants.

After washing the magnetic beads to which the PCR reaction product was bound twice with 70% (v/v) ethanol, DNA bound to the magnetic beads was eluted with 40 μL of TE (Tris-EDTA) buffer solution.

<<DNA Sequencing>>

(1) Preparation of Sequence Library Mix

In order to perform dual index sequencing using a next generation sequencer (Miseq, Illumina Co.), two kinds of adapters including a flow cell binding sequence (P5 sequence or P7 sequence), an index sequence for sample identification, and a sequence primer binding sequence were added to both ends of a DNA fragment obtained by multiplex PCR, respectively.

Specifically, addition of sequences was performed to both ends by performing PCR using the Multiplex PCR Assay kit and using 1.25 μM of each of primers D501-F and D701-R to D706-R (shown in Table 12). As PCR conditions, 5 cycles of thermal cycle: [thermal denaturation (94°C, 45 seconds) - annealing (50°C, 60 seconds) - extension (72°C, 30 seconds)] were carried out after initial thermal denaturation (94°C, 3 minutes), and then 11 cycles of thermal cycle: [thermal denaturation (94°C, 45 seconds) - annealing (55°C, 60 seconds) - extension (72°C, 30 seconds)] were carried out.

[Table 12]

| Primer | | | |
|---|---|---|---|
| Name | Base sequence (5' →3') | Size (mer) | SEQ ID NO |
| D501-F | AATGATACGGCGACCACCGAGATCTACACtatagcctTCTTTCCCTACACGACGCTCTTCCGATCTCTG | 69 | 51 |
| D701-R | CAAGCAGAAGACGGCATACGAGATcgagtaatGTGACTGGAGTTCAGACGTGTGCTCTTCCGATCTGAC | 69 | 52 |
| D702-R | CAAGCAGAAGACGGCATACGAGATtctccggaGTGACTGGAGTTCAGACGTGTGCTCTTCCGATCTGAC | 69 | 53 |
| D703-R | CAAGCAGAAGACGGCATACGAGATaatgagcgGTGACTGGAGTTCAGACGTGTGCTCTTCCGATCTGAC | 69 | 54 |
| D704-R | CAAGCAGAAGACGGCATACGAGATggaatctcGTGACTGGAGTTCAGACGTGTGCTCTTCCGATCTGAC | 69 | 55 |
| D705-R | CAAGCAGAAGACGGCATACGAGATttctgaatGTGACTGGAGTTCAGACGTGTGCTCTTCCGATCTGAC | 69 | 56 |
| D706-R | CAAGCAGAAGACGGCATACGAGATacgaattcGTGACTGGAGTTCAGACGTGTGCTCTTCCGATCTGAC | 69 | 57 |

The obtained PCR product was purified using Agencourt AMPure XP (Beckman Coulter, Inc.), and the DNA was quantitatively determined using KAPA Library Quantification Kits (manufactured by Japan Genetics Co., Ltd.).

(Sequence Analysis of Amplification Product)

The prepared sequence library mix was sequenced using Miseq Reagent Kit v2 300 Cycle (manufactured by Illumina), and therefore a FastQ file was obtained.
After mapping a human genome sequence (hg19) from the obtained FastQ data using BWA (Burrows-Wheeler Aligner), gene polymorphism information was extracted by SAMtools, and the number of sequence reading of each detection region was calculated by BEDtools.

(Confirmation of Cell Information)

The result of sequence analysis is shown in Table 13.

[Table 13]

| No. | Gene region | Number of reading for 1 cell | Number of reading for 10 cells | Number of reading for 100 cells |
|---|---|---|---|---|
| 1 | ALK_1 | 22822 | 13766 | 18390 |
| 2 | ALK_2 | 16423 | 15372 | 10970 |
| 3 | ALK_3 | 9465 | 6701 | 4558 |
| 4 | FGFR3_1 | 2506 | 356 | 902 |
| 5 | FGFR3_2 | 1624 | 1736 | 624 |
| 6 | FGFR3_3 | 4665 | 2787 | 678 |
| 7 | PDGFRA_1 | 14152 | 9991 | 7556 |
| 8 | PDGFRA_2 | 19503 | 21875 | 7435 |
| 9 | PDGFRA_3 | 8927 | 15103 | 9621 |
| 10 | KIT_1 | 2307 | 1570 | 2077 |
| 11 | KIT_2 | 12523 | 12188 | 8666 |
| 12 | KIT_3 | 11561 | 11566 | 4446 |
| 13 | FGFR4_1 | 3729 | 5204 | 1632 |
| 14 | FGFR4_2 | 3729 | 5204 | 1632 |
| 15 | EGFR_1 | 15098 | 9941 | 10952 |
| 16 | EGFR_2 | 12042 | 12226 | 15052 |
| 17 | MET_1 | 31702 | 15617 | 18079 |
| 18 | FGFR1_1 | 36722 | 40340 | 18217 |
| 19 | FGFR2_1 | 12520 | 12088 | 8131 |
| 20 | FGFR2_2 | 19535 | 11371 | 11886 |

In the single cell, the number of reading could be detected in all 20 gene regions. Therefore, it became clear that using this method enables the amplification of exactly 20 gene regions from a single cell.
In addition, even in 10 cells and 100 cells, the number of reading could be detected in all 20 gene regions.
In addition, Table 14 shows detected base information in mutation numbers.

[Table 14]

| Mutation No. | Gene name | Chromosome | Coordinate | 1 Cell | 10 Cells | 100 Cells |
|---|---|---|---|---|---|---|
| 1 | ALK | 2 | 29222392 | C | C | C |
| 2 | ALK | 2 | 29220829 | G | G | G |
| 3 | ALK | 2 | 29220747 | C | C | C |
| 4 | ALK | 2 | 29209816 | C | C | C |
| 5 | ALK | 2 | 29220765 | G | G | G |
| 6 | ALK | 2 | 29209798 | C | C | C |
| 7 | ALK | 2 | 29222405 | G | G | G |
| 8 | EGFR | 7 | 55181378 | C | C | C |
| 9 | EGFR | 7 | 55191822 | T | T | T |
| 10 | EGFR | 7 | 55191831 | T | T | T |
| 11 | FGFR1 | 8 | 38416042 | A | A | A |
| 12 | FGFR2 | 10 | 121496705 | C | C | C |
| 13 | FGFR2 | 10 | 121498522 | T | T | T |
| 14 | FGFR3 | 4 | 1806604 | G | G | G |
| 15 | FGFR3 | 4 | 1806162 | A | A | A |
| 16 | FGFR3 | 4 | 1805767 | G | G | G |
| 17 | FGFR4 | 5 | 177095415 | C | C | C |
| 18 | FGFR4 | 5 | 177095550 | G | G | G |
| 19 | KIT | 4 | 54733155 | A | A | A |
| 20 | KIT | 4 | 54729353 | C | C | C |
| 21 | KIT | 4 | 54727447 | T | T | T |
| 22 | MET | 7 | 116783374 | T | T | T |
| 23 | MET | 7 | 116783353 | G | G | G |
| 24 | MET | 7 | 116783419 | A | A | A |
| 25 | PDGFRA | 4 | 54285926 | A | A | A |
| 26 | PDGFRA | 4 | 54278380 | C | C | C |
| 27 | PDGFRA | 4 | 54274869 | T | T | T |

Bases detected from a single cell were confirmed to the same as bases detected from 10 cells and 100 cells in that these are equivalent at all mutation points.

Based on these results, it became clear that it is possible to accurately detect mutation sites existing in a plurality of gene regions from a single cell.

[Comparative Example 2]

Primer pairs in which complementarity between primers is not considered were designed instead of primer pairs which were used in Example 2 and designed such that complementarity between primers is reduced, for comparison for checking the effect of calculating the complementarity. In regard to designing of primers, each of 20 bp primers of which a Tm value was 56°C to 64°C and a PCR amplification base length was 140 to 180 base pairs was prepared using Primer 3 so as to detect the same mutation or gene region as that of Example 2.

Sequence determination of the amplification products was performed in the same manner as in Example 2 except that multiplex PCR was performed using these primer pairs. As a result, regions in which the number of reading of each gene region of the next generation sequence data was not detected were increased, and it was confirmed that it is not possible to amplify all of the gene regions.
[Sequence List] International Application W-6025PCT Base Sequence of Single Cell Derived from Vertebrate JP17032110 20170906----00210309951701876096 Normal 20170906112615201707310924081230 P1AP101 W-14.app Based on International Patent Cooperation Treaty

## Claims

1. A method for obtaining base sequence information of a single cell derived from a vertebrate, comprising:

   an objective region selection step of selecting at least one objective region for obtaining base sequence information, from regions on vertebrate genomic DNA;
   a single cell isolation step of isolating a single cell from a biological sample derived from the vertebrate;
   a genomic DNA extraction step of extracting genomic DNA from the single cell;
   a PCR amplification step of PCR amplifying the at least one objective region by using a primer set that is designed to PCR amplify the at least one objective region and using genomic DNA extracted in the genomic DNA extraction step as a template; and
   a DNA sequencing step of decoding a DNA base sequence of a PCR amplification product obtained in the PCR amplification step so as to obtain the base sequence information of the at least one objective region,
   wherein the objective region selection step, and steps from the single cell isolation step to the genomic DNA extraction step are performed in random order, and
   wherein the primer set that is designed to PCR amplify the at least one objective region is designed through a method for designing a primer set used for a polymerase chain reaction, the designing method including:

      a target region selection step a) of selecting a target region from the at least one objective region;
      a primer candidate base sequence generation step b) of generating at least one base sequence of a primer candidate for PCR amplifying the target region based on each base sequence in each of vicinity regions at both ends of the target region on the vertebrate genomic DNA;
      a local alignment step c) of obtaining a local alignment score by performing pairwise local alignment on two base sequences included in each of combinations which are obtainable by selecting base sequences of two primer candidates from the base sequences of the primer candidates generated in the primer candidate base sequence generation step, under a condition that partial sequences to be compared have 3' terminal of the two base sequences;
      a first stage selection step d) of performing first stage selection of the base sequence of the primer candidate for PCR amplifying the target region based on the local alignment score;
      a global alignment step e) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first stage selection step;
      a second stage selection step f) of performing second stage selection of the base sequence of the primer candidate for PCR amplifying the target region based on the global alignment score; and
      a primer employment step g) of employing the base sequence of the primer candidate which is selected in both of the first stage selection step and the second stage selection step as the base sequence of the primer for PCR amplifying the target region,
      wherein both steps of the local alignment step and the first stage selection step, and both steps of the global alignment step and the second stage selection step are performed in random order or at the same time.

2. A method for obtaining base sequence information of a single cell derived from a vertebrate, comprising:

   an objective region selection step of selecting at least one objective region for obtaining base sequence information, from regions on vertebrate genomic DNA;
   a single cell isolation step of isolating a single cell from a biological sample derived from the vertebrate;
   a genomic DNA extraction step of extracting genomic DNA from the single cell;
   a PCR amplification step of PCR amplifying the at least one objective region by using a primer set that is designed to PCR amplify the at least one objective region and using genomic DNA extracted in the genomic DNA extraction step as a template; and
   a DNA sequencing step of decoding a DNA base sequence of a PCR amplification product obtained in the PCR amplification step so as to obtain the base sequence information of the at least one objective region,
   wherein the objective region selection step, and steps from the single cell isolation step to the genomic DNA extraction step are performed in random order, and
   wherein the primer set that is designed to PCR amplify the at least one objective region is designed through a method for designing a primer set used for a polymerase chain reaction, the designing method including:

a first step of target region selection $a_1$) of selecting a first target region from the at least one objective region;
a first step of primer candidate base sequence generation $b_1$) of generating at least one base sequence of a primer candidate for PCR amplifying the first target region based on each base sequence in each of vicinity regions at both ends of the first target region on the vertebrate genomic DNA;
a first step of local alignment $c_1$) of obtaining a local alignment score by performing pairwise local alignment on two base sequences included in each of combinations which are obtainable by selecting base sequences of two primer candidates from the base sequences of the primer candidates generated in the first step of primer candidate base sequence generation, under a condition that partial sequences to be compared have 3' terminal of the two base sequences;
a first step of first stage selection $d_1$) of performing first stage selection of the base sequence of the primer candidate for PCR amplifying the first target region based on the local alignment score;
a first step of global alignment $e_1$) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first step of first stage selection;
a first step of second stage selection $f_1$) of performing second stage selection of the base sequence of the primer candidate for PCR amplifying the first target region based on the global alignment score;
a first step of primer employment $g_1$) of employing the base sequence of the primer candidate which is selected in both of the first step of first stage selection and the first step of second stage selection as the base sequence of the primer for PCR amplifying the first target region;
a second step of target region selection $a_2$) of selecting a second target region from objective regions which have not yet been selected from the at least one objective region;
a second step of primer candidate base sequence generation $b_2$) of generating at least one base sequence of a primer candidate for PCR amplifying the second target region based on each base sequence in each of vicinity regions at both ends of the second target region on the vertebrate genomic DNA;
a second step of local alignment $c_2$) of obtaining a local alignment score by performing pairwise local alignment on two base sequences included in each of combinations which are combinations obtainable by selecting base sequences of two primer candidates from the base sequences of the primer candidates generated in the second step of primer candidate base sequence generation and base sequences of a primer already employed, and combinations obtainable by selecting a base sequence of one primer candidate and a base sequence of one primer already employed, under a condition that partial sequences to be compared have 3' terminal of the two base sequences;
a second step of first stage selection $d_2$) of performing first stage selection of the base sequence of the primer candidate for PCR amplifying the second target region based on the local alignment score;
a second step of global alignment $e_2$) of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second step of first stage selection and base sequences of a primer already employed, and combinations obtainable by selecting a base sequence of one primer candidate and a base sequence of one primer already employed;
a second step of second stage selection $f_2$) of performing second stage selection of the base sequence of the primer candidate for PCR amplifying the second target region based on the global alignment score; and
a second step of primer employment $g_2$) of employing the base sequence of the primer candidate which is selected in both of the second step of first stage selection and the second step of second stage selection as a base sequence of a primer for PCR amplifying the second target region,
wherein both steps of the first step of local alignment and the first step of first stage selection, and both steps of the first step of global alignment and the first step of second stage selection are performed in random order or at the same time,
wherein both steps of the second step of local alignment and the second step of first stage selection, and both steps of the second step of global alignment and the second step of second stage selection are performed in random order or at the same time, and
wherein in a case where the at least one objective region has three or more objective regions, and in case of employing a base sequence of a primer for PCR amplifying third and subsequent target regions, which have not yet been selected from the three or more objective regions, each step from the second step of target region selection to the second step of primer employment is repeated for the third and subsequent target regions.

3. A method for obtaining base sequence information of a single cell derived from a vertebrate, comprising:

an objective region selection step of selecting at least one objective region for obtaining base sequence information, from regions on vertebrate genomic DNA;
a single cell isolation step of isolating a single cell from a biological sample derived from the vertebrate;
a genomic DNA extraction step of extracting genomic DNA from the single cell;
a PCR amplification step of PCR amplifying the at least one objective region by using a primer set that is designed to PCR amplify the at least one objective region and using genomic DNA extracted in the genomic DNA extraction step as a template; and
a DNA sequencing step of decoding a DNA base sequence of a PCR amplification product obtained in the PCR amplification step so as to obtain the base sequence information of the at least one objective region,
wherein the objective region selection step, and steps from the single cell isolation step to the genomic DNA extraction step are performed in random order, and
wherein the primer set that is designed to PCR amplify the at least one objective region is designed through a method for designing a primer set used for a polymerase chain reaction, the designing method including:

a target region multiple selection step a-0) of selecting a plurality of target regions from the at least one objective region;
a primer candidate base sequence multiple generation step b-0) of generating at least one base sequence of a primer candidate for PCR amplifying the plurality of target regions based on each base sequence in each of vicinity regions at both ends of the plurality of target regions on the vertebrate genomic DNA;
a first local alignment step c-1) of obtaining a local alignment score by performing pairwise local alignment on two base sequences included in each of combinations which are obtainable by selecting base sequences of two primer candidates from the base sequences of the primer candidates for PCR amplifying the first target region among the base sequences of the primer candidates generated in the primer candidate base sequence multiple generation step, under a condition that partial sequences to be compared have 3' terminal of the two base sequences;
a first first-stage selection step d-1) of performing first stage selection of the base sequence of the primer candidate for PCR amplifying the first target region based on the local alignment score;
a first global alignment step e-1) of obtaining a global alignment score by performing pairwise global alignment on a base sequence, which has a predetermined sequence length and has 3' terminal of two base sequences included in the combinations, in each of combinations which are obtainable by selecting base sequences of two primer candidates from the base sequences of the primer candidates selected in the first first-stage selection step;
a first second-stage selection step f-1) of performing second stage selection of the base sequence of the primer candidate for PCR amplifying the first target region based on the global alignment score;
a first primer employment step g-1) of employing the base sequence of the primer candidate which is selected in both of the first first-stage selection step and the first second-stage selection step as the base sequence of the primer for PCR amplifying the first target region;
a second local alignment step c-2) of obtaining a local alignment score by performing pairwise local alignment on two base sequences included in each of combinations which are combinations obtainable by selecting base sequences of two primer candidates from the base sequences of the primer candidates for PCR amplifying the second target region among the base sequences of the primer candidates generated in the primer candidate base sequence multiple generation step and base sequences of a primer already employed, and combinations obtainable by selecting a base sequence of one primer candidate and a base sequence of one primer already employed, under a condition that partial sequences to be compared have 3' terminal of the two base sequences;
a second first-stage selection step d-2) of performing first stage selection of the base sequence of the primer candidate for PCR amplifying the second target region based on the local alignment score;
a second global alignment step e-2) of obtaining a global alignment score by performing pairwise global alignment on a base sequence, which has a predetermined sequence length and has 3' terminal of two base sequences included in the combinations, in each of combinations which are combinations obtainable by selecting base sequences of two primer candidates from the base sequences of the primer candidates selected in the second first-stage selection step and base sequences of a primer already employed, and combinations obtainable by selecting a base sequence of one primer candidate and a base sequence of one primer already employed;
a second second-stage selection step f-2) of performing second stage selection of the base sequence of the primer candidate for PCR amplifying the second target region based on the global alignment score; and

a second primer employment step g-2) of employing the base sequence of the primer candidate which is selected in both of the second first-stage selection step and the second second-stage selection step as the base sequence of the primer for PCR amplifying the second target region,
wherein both steps of the first local alignment step and the first first-stage selection step, and both steps of the first global alignment step and the first second-stage selection step are performed in random order or at the same time,
wherein both steps of the second local alignment step and the second first-stage selection step, and both steps of the second global alignment step and the second second-stage selection step are performed in random order or at the same time, and
wherein in a case where the at least one objective region has three or more objective regions, three or more target regions are selected in the target region multiple selection step, and a base sequence of a primer candidate for PCR amplifying each of the three or more target regions is generated in the primer candidate base sequence multiple generation step, and in case of employing a base sequence of a primer for PCR amplifying third and subsequent target regions, each step from the second local alignment step to the second primer employment step is repeated for the third and subsequent target regions.

**4.** The method for obtaining base sequence information of a single cell derived from a vertebrate according to any one of claims 1 to 3,
wherein the single cell is a rare cell.

**5.** The method for obtaining base sequence information of a single cell derived from a vertebrate according to claim 4, wherein the rare cell is a nucleated red blood cell derived from a fetus.

**6.** The method for obtaining base sequence information of a single cell derived from a vertebrate according to claim 4, wherein the rare cell is a cancer cell.

**7.** The method for obtaining base sequence information of a single cell derived from a vertebrate according to claim 6, wherein the cancer cell is a circulating cancer cell.

**8.** The method for obtaining base sequence information of a single cell derived from a vertebrate according to any one of claims 1 to 3,
wherein the single cell is a cancer cell derived from a solid cancer of an organ.

FIG. 1

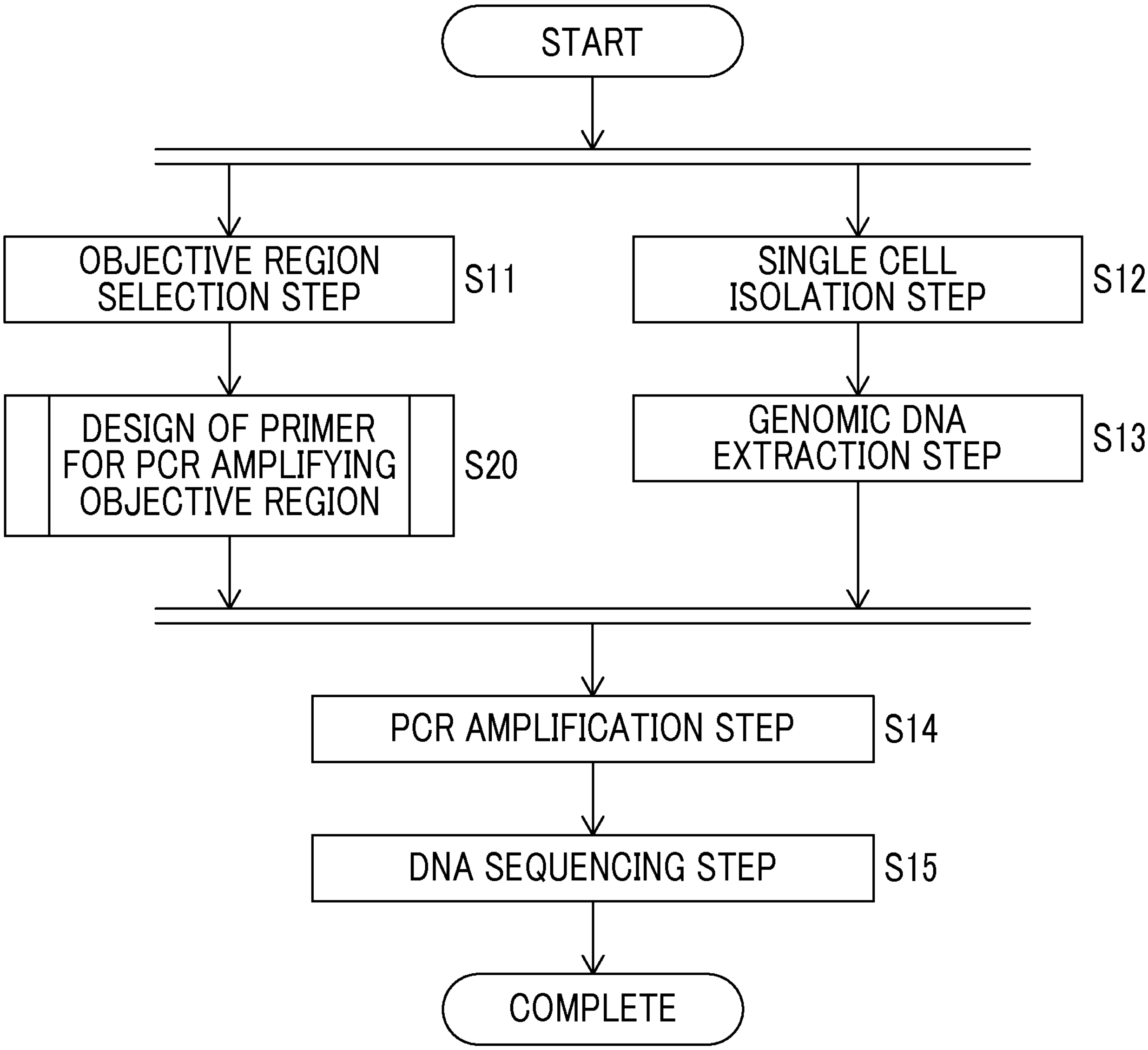
START
OBJECTIVE REGION SELECTION STEP
S11
SINGLE CELL ISOLATION STEP
S12
DESIGN OF PRIMER FOR PCR AMPLIFYING OBJECTIVE REGION
S20
GENOMIC DNA EXTRACTION STEP
S13
PCR AMPLIFICATION STEP
S14
DNA SEQUENCING STEP
S15
COMPLETE

FIG. 2

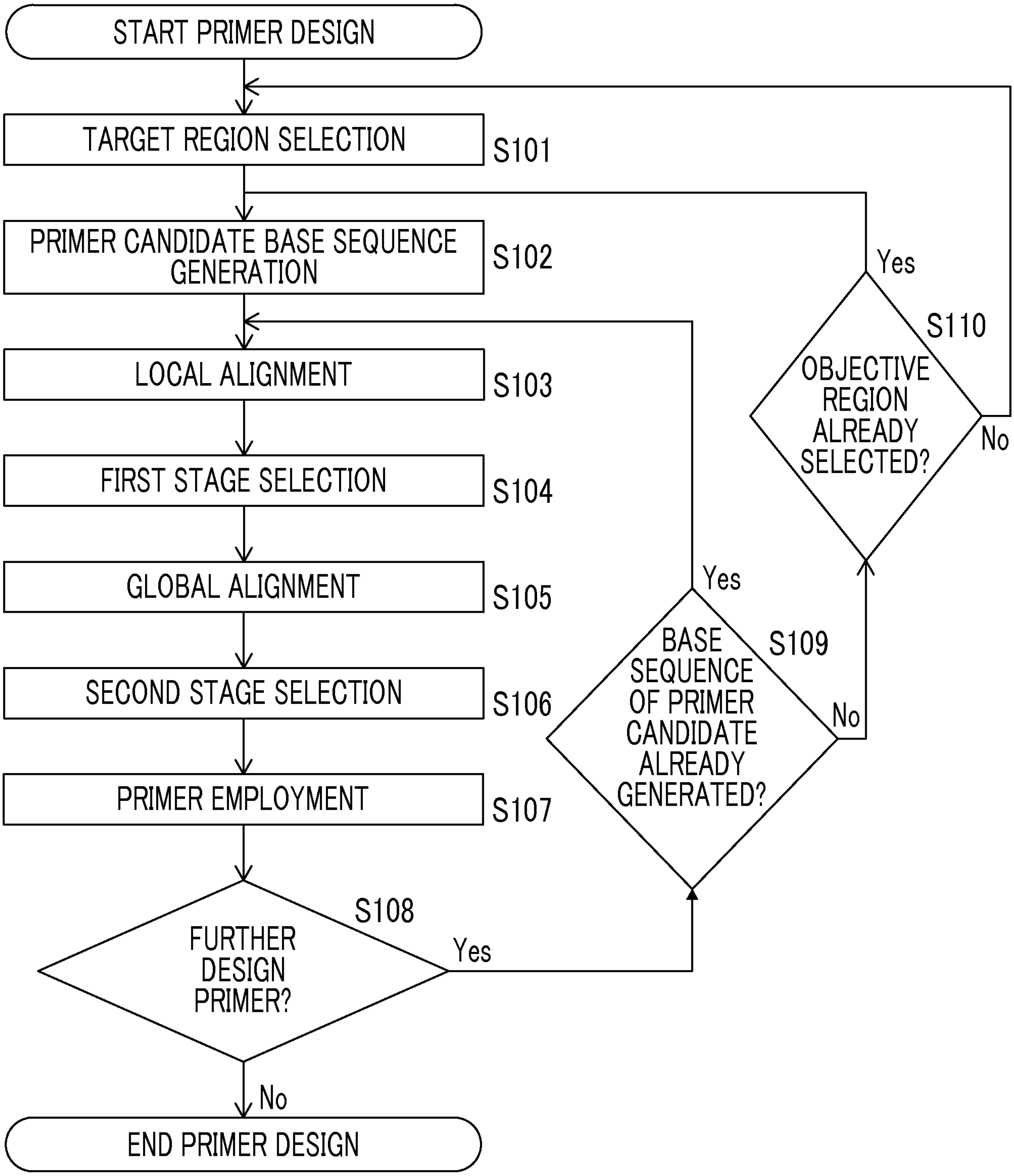
START PRIMER DESIGN
TARGET REGION SELECTION
S101
PRIMER CANDIDATE BASE SEQUENCE GENERATION
S102
LOCAL ALIGNMENT
S103
FIRST STAGE SELECTION
S104
GLOBAL ALIGNMENT
S105
SECOND STAGE SELECTION
S106
PRIMER EMPLOYMENT
S107
FURTHER DESIGN PRIMER?
S108
Yes
No
END PRIMER DESIGN
BASE SEQUENCE OF PRIMER CANDIDATE ALREADY GENERATED?
S109
Yes
No
OBJECTIVE REGION ALREADY SELECTED?
S110
Yes
No

FIG. 3

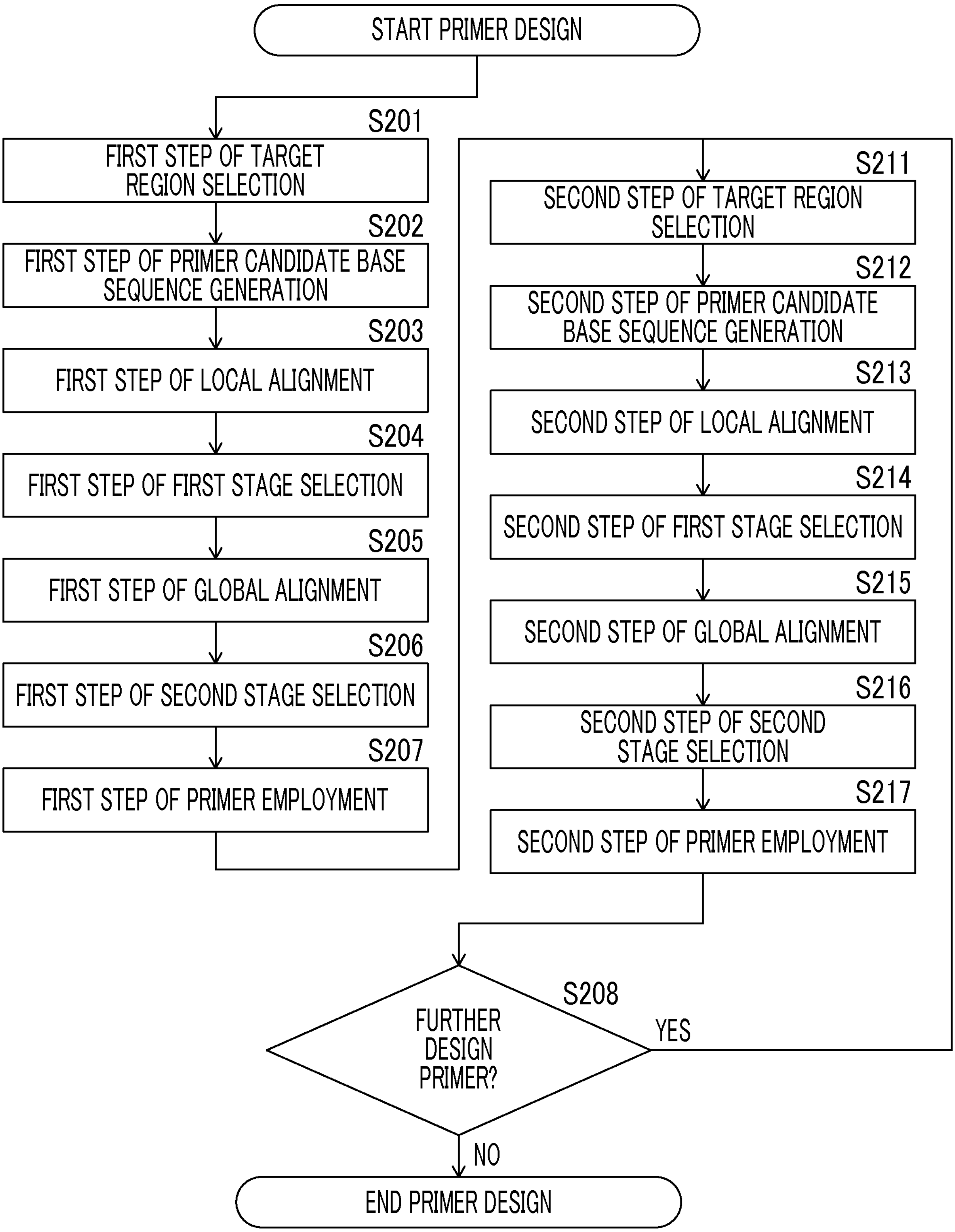
START PRIMER DESIGN
S201
FIRST STEP OF TARGET REGION SELECTION
S202
FIRST STEP OF PRIMER CANDIDATE BASE SEQUENCE GENERATION
S203
FIRST STEP OF LOCAL ALIGNMENT
S204
FIRST STEP OF FIRST STAGE SELECTION
S205
FIRST STEP OF GLOBAL ALIGNMENT
S206
FIRST STEP OF SECOND STAGE SELECTION
S207
FIRST STEP OF PRIMER EMPLOYMENT
S211
SECOND STEP OF TARGET REGION SELECTION
S212
SECOND STEP OF PRIMER CANDIDATE BASE SEQUENCE GENERATION
S213
SECOND STEP OF LOCAL ALIGNMENT
S214
SECOND STEP OF FIRST STAGE SELECTION
S215
SECOND STEP OF GLOBAL ALIGNMENT
S216
SECOND STEP OF SECOND STAGE SELECTION
S217
SECOND STEP OF PRIMER EMPLOYMENT
S208
FURTHER DESIGN PRIMER?
YES
NO
END PRIMER DESIGN

FIG. 4

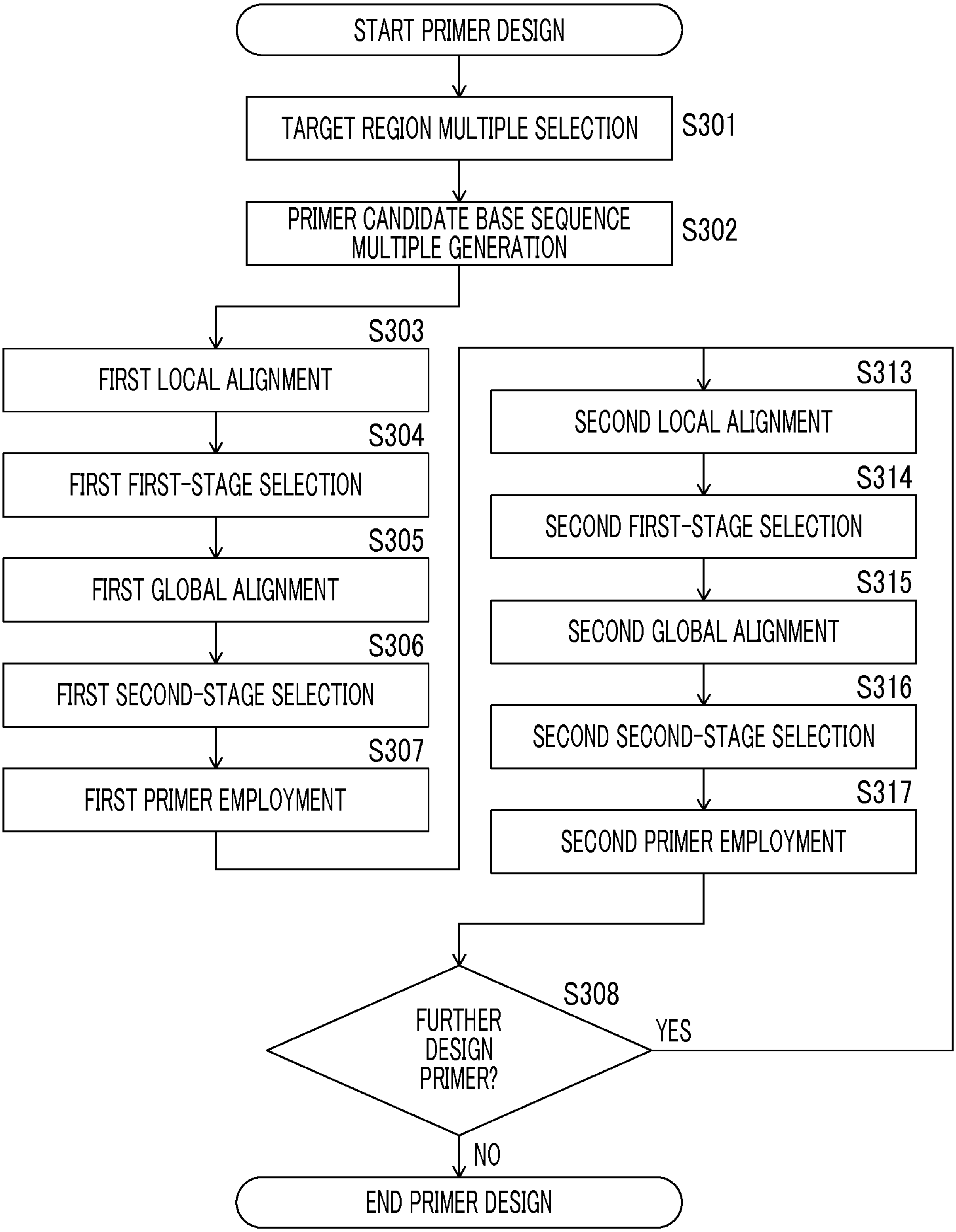
START PRIMER DESIGN
TARGET REGION MULTIPLE SELECTION
S301
PRIMER CANDIDATE BASE SEQUENCE MULTIPLE GENERATION
S302
S303
FIRST LOCAL ALIGNMENT
S304
FIRST FIRST-STAGE SELECTION
S305
FIRST GLOBAL ALIGNMENT
S306
FIRST SECOND-STAGE SELECTION
S307
FIRST PRIMER EMPLOYMENT
S313
SECOND LOCAL ALIGNMENT
S314
SECOND FIRST-STAGE SELECTION
S315
SECOND GLOBAL ALIGNMENT
S316
SECOND SECOND-STAGE SELECTION
S317
SECOND PRIMER EMPLOYMENT
S308
FURTHER DESIGN PRIMER?
YES
NO
END PRIMER DESIGN

FIG. 5

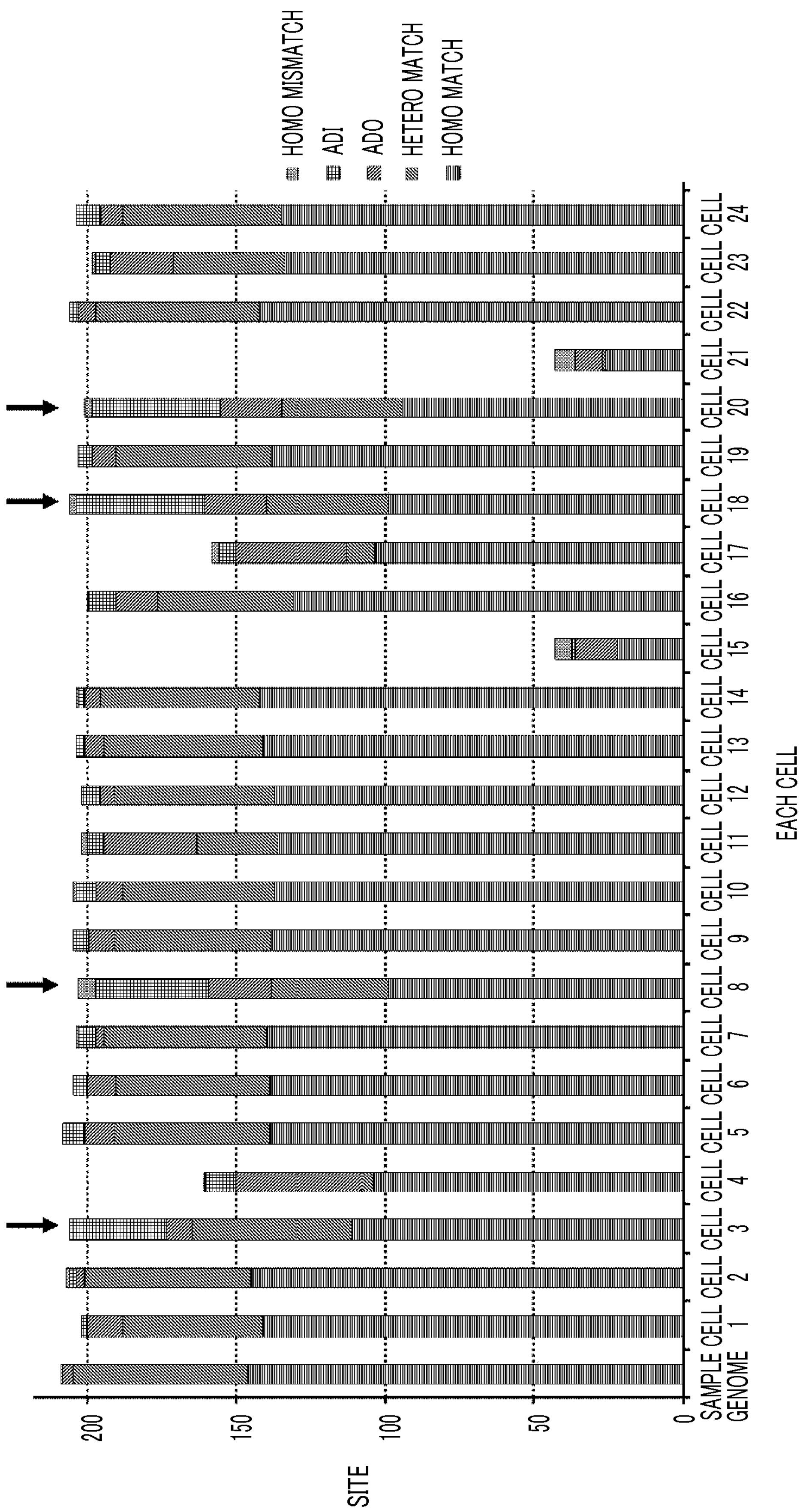
HOMO MISMATCH
ADI
ADO
HETERO MATCH
HOMO MATCH
SITE
0
50
100
150
200
SAMPLE GENOME
CELL 1
CELL 2
CELL 3
CELL 4
CELL 5
CELL 6
CELL 7
CELL 8
CELL 9
CELL 10
CELL 11
CELL 12
CELL 13
CELL 14
CELL 15
CELL 16
CELL 17
CELL 18
CELL 19
CELL 20
CELL 21
CELL 22
CELL 23
CELL 24
EACH CELL

FIG. 6

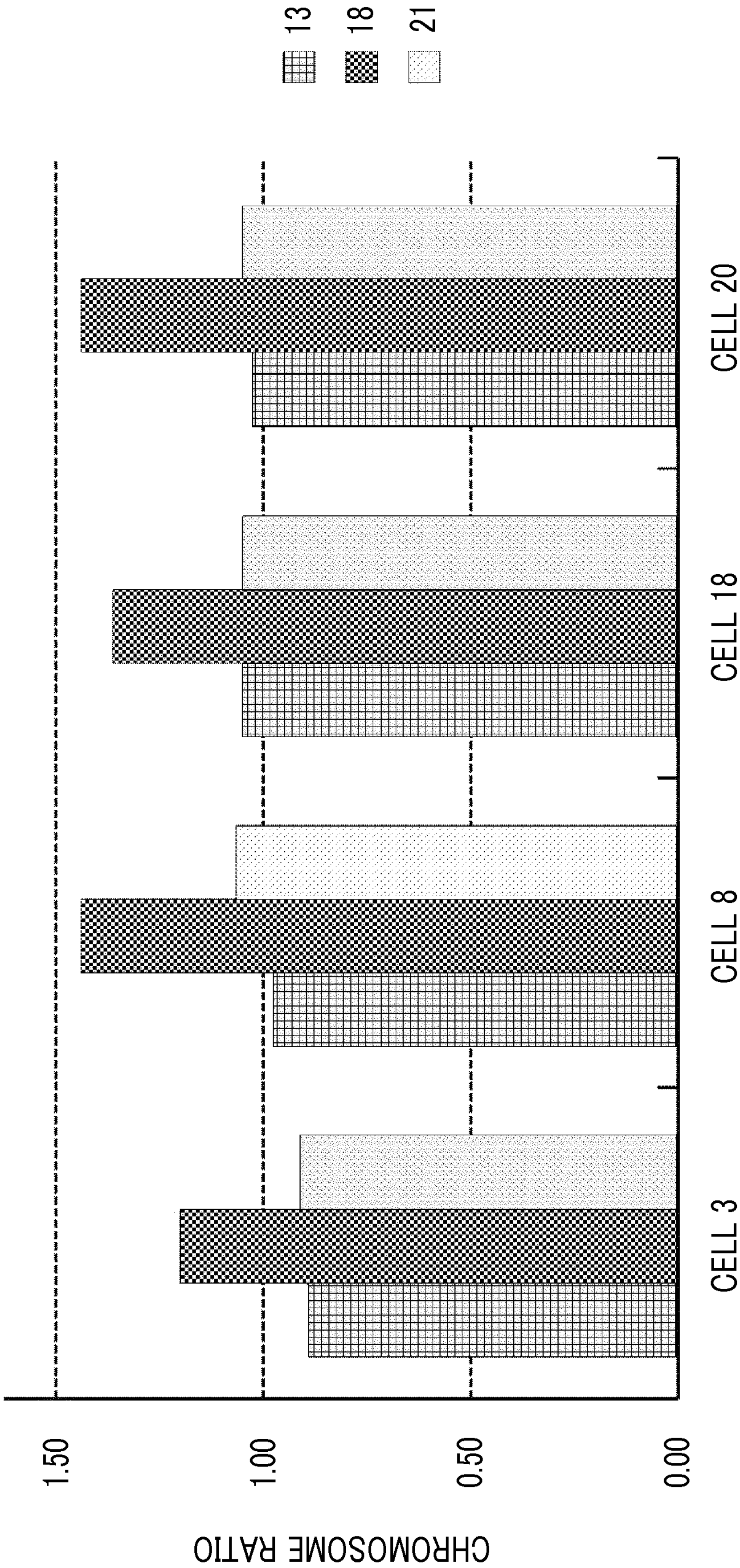
13
18
21
CHROMOSOME RATIO
1.50
1.00
0.50
0.00
CELL 3
CELL 8
CELL 18
CELL 20

**INTERNATIONAL SEARCH REPORT**

International application No.
PCT/JP2017/032110

A. CLASSIFICATION OF SUBJECT MATTER
Int.Cl. C12Q1/68(2006.01)i, C12Q1/02(2006.01)i, C12N15/09(2006.01)n

According to International Patent Classification (IPC) or to both national classification and IPC

B. FIELDS SEARCHED
Minimum documentation searched (classification system followed by classification symbols)
Int.Cl. C12Q1/68, C12Q1/02, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model specifications of Japan | 1994-2017 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2016/042830 A1 (FUJIFILM CORP.) 24 March 2016, claims, paragraphs [0038]-[0067] (Family: none) | 1-5<br>6-8 |
| Y<br>A | NOVAK, R. et al., Single-Cell Multiplex Gene Detection and Sequencing with Microfluidically Generated Agarose Emulsions, Angewandte Chemie International Edition, 2011, vol. 50, no. 2, pp. 390-395, in particular, page 391 | 1-4, 6-8<br>5 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 29 November 2017 (29.11.2017) | 12 December 2017 (12.12.2017) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2017/032110

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | PONTEN, F. et al., Genomic analysis of single cells from human basal cell cancer using laser-assisted capture microscopy, Mutation Research Genomics, 1997, vol. 382, pp. 45-55, all. in particular, pp. 46-47 | 1-4, 6-8<br>5 |
| Y | KAPLINSKI, L. and REMM, M., MultiPLX: Automatic grouping and evaluation of PCR primers, PCR Primer Design, Second Edition, 2015, pp. 127-142, in particular, pp. 128-132 | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004081225 A
- WO 2008004691 A
- WO 2012023298 A
- JP 2011163830 A
- JP 2013042689 A
- JP 17032110 A
- JP 20170906 A


### Non-patent literature cited in the description


- **LI, H. et al.** Fast and accurate short read alignment with Burrows-Wheeler transform. *Bioinformatics,* 2009, vol. 25 (14), 1754-1760
- **LI, H. et al.** Fast and accurate long-read alignment with Burrows-Wheeler transform. *Bioinformatics,* 2010, vol. 26 (5), 589-595
- **LI, HENG et al.** The Sequence Alignment / Map format and SAMtools. *Bioinformatics,* 2009, vol. 25 (16), 2078-2079
- **QUINLAN, A. R. et al.** BEDTools: a flexible suite of utilities for comparing genomic features. *Bioinformatics,* 2010, vol. 26 (6), 841-842
- **ALTSCHUL, S. A. et al.** Basic Local Alignment Search Tool. *Journal of Molecular Biology,* October 1990, vol. 215, 403-410
- Improved tools for biological sequence comparison. **PEARSON, W. R. et al.** Proceedings of the National Academy of Sciences of the United States of America. National Academy of Sciences, April 1988, vol. 85, 2444-2448